(19) Europäisches Patentamt
European Patent Office
Office européen des brevets

(11) Veröffentlichungsnummer: **0 265 865 B1**

(12) **EUROPÄISCHE PATENTSCHRIFT**

(45) Veröffentlichungstag der Patentschrift: **03.06.92**

(51) Int. Cl.$^5$: **A61K 37/14**

(21) Anmeldenummer: **87115562.8**

(22) Anmeldetag: **23.10.87**

(54) **Blutersatzmittel.**

(30) Priorität: **28.10.86 DE 3636590**

(43) Veröffentlichungstag der Anmeldung:
**04.05.88 Patentblatt 88/18**

(45) Bekanntmachung des Hinweises auf die Patenterteilung:
**03.06.92 Patentblatt 92/23**

(84) Benannte Vertragsstaaten:
**AT BE CH DE ES FR GB GR IT LI LU NL SE**

(56) Entgegenhaltungen:
**EP-A- 0 140 640**
**WO-A-86/04240**

(73) Patentinhaber: **B. Braun Melsungen AG**
**Carl-Braun Strasse**
**W-3508 Melsungen(DE)**

(72) Erfinder: **Feller, Wolfgang, Dr.**
**Ernstbergstrasse 30**
**W-3508 Melsungen(DE)**
Erfinder: **Macht, Norbert**
**Hessenwinkel 26**
**W-3508 Melsungen(DE)**
Erfinder: **Böttrich, Johannes, Dr.**
**Talstrasse 5**
**W-6322 Kirtorf-Lehrbach(DE)**

(74) Vertreter: **von Kreisler, Alek, Dipl.-Chem. et al**
**Deichmannhaus am Hauptbahnhof**
**W-5000 Köln 1(DE)**

**EP 0 265 865 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Hämoglobin-enthaltende Blutersatzmittel sowie Verfahren zu deren Herstellung und Verwendung als Arzneimittel.

Menschliche oder tierische Hämoglobin-enthaltende, reversibel Sauerstoff aufnehmende oder abgebende wäßrige Lösungen werden seit vielen Jahren weltweit daraufhin untersucht, ob sie als Volumenersatzmittel mit sauerstoffübertragender Funktion die durch massiven Blutverlust bedingte Ischämie zu vermindern oder auszugleichen in der Lage sind.

Wäßrige Hämoglobinlösungen könnten sich bei erster Betrachtung als ideale Plasmaexpander mit kolloid-osmotischen, Sauerstoff-und Kohlendioxid-transportierenden Eigenschaften eignen. Darüberhinaus ist von reinen Hämoglobinlösungen zu erwarten, daß sie keine Blutgruppeneigenschaften aufweisen, so daß an sich beste Voraussetzungen für eine generelle Anwendung an Menschen bestehen sollten.

Rabiner et al. zeigten bereits 1967 (J. Exper. Med. 126, 1127) in tierexperimentellen Untersuchungen, daß stromafreie Hämoglobinlösungen ohne nachteilige Beeinträchtigung der Nierenfunktion und Beeinflussung des Gerinnungssystems infundiert werden können. Voraussetzung für diese gute Verträglichkeit war die Abtrennung des Lipid-enthaltenden "Stromas", der Zellmembranbruchstücke der Erythrozytenmembran, die die früher beobachtete Nephrotoxizität verursachte.

Es zeigte sich jedoch später, daß im wesentlichen zwei entscheidende Eigenschaften der stroma-freien Human-Hänoglobinlösungen deren medizinischem Einsatz im Wege stehen:
Die intravasale Halbwertszeit, d.h. die Zeit, in der die Hämoglobinkonzentration nach einer intravenösen Applikation auf 50 % des Ausgangswertes abfällt, ist sehr kurz und liegt, abhängig von der Dosierung, bei nur zwei bis vier Stunden.

Die Ursache dafür liegt in der molekularen Struktur von Human-Hämoglobin begründet. Natives Hämoglobin mit einem Molekulargewicht 64 500 besteht aus vier Untereinheiten der Tetramer-Struktur $\alpha_2\beta_2$, die unter physiologischen Bedingungen in ihrer oxygenierten Form (R = Relaxed-state) im Gleichgewicht mit ca. 4 % eines Dimeren $\alpha\beta$ mit dem Molekulargewicht von etwa 32 000 stehen. Diese Hämoglobindimeren können aufgrund ihres geringen Molekulargewichtes über die Niere ausgeschieden werden.

Der Halbsättigungsdruck (P 50-Wert) des reinen stromafreien Hämoglobins, der als Maß für das Sauerstoffbindungsverhalten zu betrachten ist, sinkt auf etwa 0,40 kPa (3 mmHg) bis 0,93 kPa (7 mmHg) Sauerstoffpartialdruck ab. Der Sauerstoffpartialdruck bei Halbsättigung des Hämoglobins in intakten Erythrozyten liegt unter physiologischen Bedingungen im Vergleich dazu bei 3,33 kPa (25 mmHg) bis 3,72 kPa (28 mmHg). Die erhöhte Sauerstoffaffinität der stromafreien Hb-Lösung hat zur Folge, daß von dem Hämoglobin in der Lunge zwar Sauerstoff aufgenommen werden kann, dieser jedoch im peripheren Gewebe bei $O_2$-Partialdrucken um 5,3 kPa (40 mmHg) nicht wieder abgegeben wird.

Abb. 1 zeigt Sauerstoffbindungskurven von Human-Erythrozyten und einer stromafreien Hb-Lösung. Die "Linksverschiebung" der Hämoglobinlösung zu geringeren $O_2$-Partialdrucken ist deutlich sichtbar. Meßbedingungen: Puffer: 225 mM BISTRIS, pH 7,4, Meßtemperatur: 37° C, Hb-Konzentration = 60 mg/ml.

Zur Überwindung dieser beiden Probleme, nämlich einer Erniedrigung der Sauerstoffaffinität und Erhöhung der intravasalen Halbwertszeit, bzw. Verweildauer, wurden in der Vergangenheit mehrere experimentelle Wege eingeschlagen, um ein stromafreies Hämoglobin ohne die genannten nachteiligen Eigenschaften herzustellen. Das Ziel war deshalb stets, ein Präparat mit den folgenden Anforderungen zu entwickeln:

1. Die intravasale Halbwertszeit (als Maß für die intravasale Verweildauer) für Hämoglobinlösungen sollte bei 10 bis 30 Stunden liegen.
2. Die stromafreie Hämoglobinlösung überträgt reversibel Sauerstoff und Kohlendioxid. Der Halbsättigungsdruck (P 50-Wert) der Sauerstoffbindungskurve einer Hämoglobinlösung sollte unter physiologischen Bedingungen mindestens bei 3,33 kPa (25 mmHg) bis 3,72 kPa (28 mmHg) liegen.
3. Die Lösung sollte onkotisch aktiv sein und kolloidosmotische Drucke von 3,33 kPa (25 mmHg) bis 4,00 kPa (30 mmHg) aufweisen.
4. Die Lösung sollte lagerstabil sein. Eine Lagerungszeit - wenn möglich ohne daß Kühlung oder gar Einfrieren erforderlich ist - von etwa einem Jahr wird angestrebt.
5. Die Lösung sollte Fließeigenschaften aufweisen, die sich an der Blutviskosität orientieren. Geringere Viskositäten als die von Blut können akzeptiert werden.
6. Die ionische Zusammensetzung der Lösung sollte eine Osmolarität von 260 bis 320 mOsm gewährleisten und sich an der ionischen Zusammensetzung von Humanblut orientieren.
7. Die Lösung sollte steril, pyrogenfrei und frei von Viruspartikeln (z.B. Hepatitis, AIDS) sein.
8. Die Lösung sollte keine Blutgruppeneigenschaften aufweisen.

9. Der Methämoglobingehalt sollte unter 5 %, bezogen auf die Gesamthämoglobinkonzentration, liegen.

10. Die Lösung sollte keine Stromabestandteile enthalten.

11. Das Sauerstoffbindungs- und -abgabeverhalten sollte den Patienten bei ausreichender Sauerstoffversorgung des peripheren Gewebes erlauben, Luft einzuatmen. Eine zusätzliche Anreicherung der Atemluft mit $O_2$ sollte nicht notwendig sein. Die Sauerstoffbindung von Human-Hämoglobin im Erythrozyten liegt um 1,34 ml Sauerstoff pro Gramm Hämoglobin. Dieser Wert sollte unter Normalbedingungen auch von der Hämoglobin-Lösung erreicht werden.

12. Die Lösung sollte möglichst wenige Hilfsstoffe in möglichst geringen Konzentrationen enthalten.

13. Die Lösung sollte preiswert, reproduzierbar und mit hoher Ausbeute - bezogen auf Hämoglobin - herstellbar sein.

14. Die Lösung muß auch in hohen Dosen verträglich sein und darf keine immunologischen Reaktionen bewirken.

15. Die Toxizität der Lösung sollte gering sein. Insbesondere darf durch den Katabolismus des intravasal applizierten Hämoglobins keine Beeinträchtigung des Reticuloendothelialen Systems oder der Nierenfunktion erfolgen. Alle Organfunktionen sollten während und nach der Anwendung der Hämoglobinlösung erhalten bleiben. Chronische Beeinträchtigungen der Organfunktion nach Anwendung der Hb-Lösung sollten aus bleiben.

Zum Verständnis der beschriebenen Verfahren zur Herstellung einer stromafreien Hämoglobinlösung ist es notwendig, die Hämoglobinstruktur näher zu erläutern:
Das menschliche Hämoglobin-Tetramer mit dem Molekulargewicht 64 500 ist aus vier nahezu identischen Untereinheiten aufgebaut. Das Hämoglobin-Tetramer $\alpha_2 \beta_2$ des Hämoglobins A (HbA), des wichtigsten und mengenmäßig mit dem höchsten Anteil im Blut normalerweise vorkommenden Hämoglobins, besteht aus je zwei identischen $\alpha$-und $\beta$-Ketten. Das Molekulargewicht der $\alpha$-Kette liegt bei 16 400, das der $\beta$-Kette bei 15 850.

Insgesamt enthält Hämoglobin A 42 Lysinreste, die einer chemischen Modifizierungsreaktion mit reaktiven Aminogruppen-spezifischen Vernetzungsmitteln - wie beispielsweise Glutardialdehyd- zugänglich sind.

Das Tetramer kann in zwei unterschiedlichen Konformationen vorliegen, der R (Relaxed)- oder T (Tense)-Form, die sich jedoch bezüglich ihrer Sauerstoffaffinität unterscheiden, wobei die T-Form eine größere Sauerstoffaffinität aufweist. Die Affinität des Humanhämoglobins für Sauerstoff wird in Erythrozyten durch 2,3 Diphosphoglycerat (2,3 DPG) reguliert. Eine Erhöhung des DPG-Gehaltes führt in Erythrozyten zu einer Erniedrigung der Sauerstoffaffinität und bewirkt deshalb eine "Rechtsverschiebung" der Sauerstoffbindungskurve. 2,3 DPG bindet an einer als Phosphatbindungsstelle oder "$\beta$-cleft" bekannten Stelle des Hämoglobinmoleküls und bewirkt auf diese Weise die Ausbildung ionischer "Bindungen", wobei die beiden Phosphatgruppen mit 4 Histidinresten des Hämoglobins und den terminalen Aminogrppen der $\beta$-Kette und die Carboxylgruppe des 2,3 DPG mit dem Lysinrest 82 der $\beta$-Kette eine Stabilisierung der "low Affinity"-Form des Hämoglobins bewirken. Andere anionische oder polyanionische Substanzen, wie etwa Inositolphosphate, insbesondere Inositolhexaphosphat, Inositolhexasulfat oder Pyridoxalphosphat, wirken in prinzipiell gleicher Weise wie 2,3 DPG mit Humanhämoglobin und setzen die Sauerstoffaffinität zum Teil in stärkerem Maß herab als 2, 3 DPG.

Die in der Literatur beschriebenen Lösungswege zur Herstellung einer Hämoglobinlösung können wie folgt grob durch die verwendeten Verfahrensprinzipien klassifiziert werden:

a) Inter- oder intramolekulare Vernetzung von Hämoglobin mit Hilfe von bi- oder polyfunktionellen reaktiven Vernetzungsmitteln.

b) Vernetzung von Hämoglobin mit Hilfe von bi- oder polyfunktionellen reaktiven Vernetzungmitteln und einem zusätzlichen Modifizierungsschritt, beispielsweise mit Pyridoxalphosphat, mit dem Ziel, die vernetzungsbedingte Erhöhung der Sauerstoffaffinität wenigstens zu kompensieren oder gar zu verbessern.

c) Kovalente Verknüpfung von Hämoglobin an wasserlösliche, physiologisch verträgliche Polymere wie Dextran, Polyvinylpyrrolidon, Hydroxyethylstärke, Inulin oder auch an Polyethylen- bzw. Polypropylenglykol.

d) Nicht kovalente (ionische) Verknüpfung von Hämoglobin an wasserlösliche, physiologisch verträgliche Polymere, die als Liganden anionische Gruppen, wie Inositolhexaphosphat, enthalten. Diese polymergebundenen Gruppen weisen eine hohe Bindungsaffinität zu Hämoglobin auf.

e) Umsetzung mit Modifizierungsreagenzien, die entweder zu einer inter- oder einer intramolekularen Vernetzung des Hämoglobintetrameren führen, und gleichzeitig eine "Rechtsverschiebung" der Sauerstoffbindungskurve bewirken. Diese Modifizierungsreagenzien enthalten sowohl reaktive funktionelle Gruppen im Molekül, die zur kovalenten Umsetzung mit funktionellen (Amino)-Gruppen des Hämoglobins in der Lage sind, als auch anionische Molekülteile, die die Sauerstoffaffinität bei Bindung an die

Phosphatbindungsstelle des Hämoglobins verringern. Als Beispiel sei 2-Nor-2-Formyl-pyridoxal-5-phosphat genannt.

f) Einschluß von Hämoglobin in Liposomen, sogenannte Hemosomen.

g) Kombination einzelner Lösungswege.

Beispielsweise werden in der DE-OS 24 17 619 Plasmaprotein-Ersatzmittel beschrieben, die Hämoglobin enthalten und deren Untereinheit mit Dialkyldicarbonsäureimidaten zu polymeren Produkten mit mittleren Molekülmassen von 68 000 bis 600 000 umgesetzt werden.

In der DE-OS 26 07 706 sind ebenfalls wasserlösliche Hämoglobinprodukte offenbart, deren Untereinheiten mittels geeigneter Vernetzungsmittel, wie z.B. Triazinen, mehrfach substituierten, mehrfunktionellen Benzolderivaten, linearen oder zyklischen mehrfunktionellen Poly-Alkylenderivaten, Dicarbonsäurederivaten oder Dialdehyden zu Polymeren mit Molekülmassen von 64 000 bis 1 000 000 D vernetzt werden.

Zur Erhöhung der intravasalen Verweilzeit einer stromafreien Hämoglobinlösung wurde auch versucht, Hämoglobin an physiologisch unbedenklich hochpolymere Substanzen kovalent zu binden. Als physiologisch verträgliche Polymere wurden insbesondere Hydroxyethylstärke (DE-OS 26 16 086) und weitere über Brücken kovalent gebundene Polysaccharide (DE-OS 30 29 307), Inulin (EP-A 81 302 858.6), Dextran [S. Thamon, J. Blumenstein und J.T. Wong, P.N.A.S. 73, 2128 (1976); R. G. Humphries et al., P.B.S., 191 (1981); J.E. Baldwin et al., Tetrahedron 37, 1723 (1981)] oder Polyethylenglykole unterschiedlicher Molekülmassen [K. Ajisaka et al., B.B. Res. Comm., 97, 1076 (1980); US-PS 4 301 144] beschrieben.

Eine wie auch immer geartete Modifizierung des Hämoglobinmoleküls bedeutet jedoch auch gleichzeitig eine Änderung seiner natürlichen Quartärstruktur und - damit verbunden - eine Beeinflussung des Sauerstoffbindungsverhaltens, indem Hillkoeffizient und P 50-Wert stark vermindert werden.

Desoxygeniertes, modifiziertes Hämoglobin kann zwar Sauerstoff binden, diesen aber wegen der erhöhten Affinität des Systems Hb/$O_2$ nicht in der benötigten Menge an das periphere Gewebe abgeben.

Dieses Phänomen läßt meßtechnisch in der Aufnahme einer Sauerstoffbindungs- (dissoziations)-kurve erfassen; die Bindungskurve des modifizierten Hämoglobins (s. Abb. 1A) ist gegenüber der des nicht chemisch veränderten Hämoglobins (s. Abb. 1B) nach links, d.h. zu niedrigen Sauerstoffpartialdrucken hin, verschoben.

Allen bisher bekannten Vernetzungsreaktionen und kovalenten Kopplungsmethoden an wasserlösliche Polymere ist gemeinsam, daß der P 50-Wert gegenüber nicht polymerisiertem Hämoglobin absinkt. Ferner wurde eine Abfall der Hämoglobin-Kooperativität beobachtet. Der Hillkoeffizient, der als ein Maß für diese Kooperativität angesehen wird, sinkt dabei von 2,8 auf Werte unter 2.

1969 beschrieben erstmals Benesch et al. den Einfluß von Pyridoxalphosphat auf die Erniedrigung der Sauerstoffaffinität von Hämoglobin. Nachdem es gelungen war, Pyridoxalphosphat chemisch an HbA zu koppeln, wurden pyridoxalierte Hämoglobine von verschiedenen Arbeitsgruppen eingesetzt, um polymerisierte und gleichzeitig pyridoxalierte Hämoglobinprodukte mit Sauerstoffhalbsättigungsdrucken von etwa 3,6 kPa (27 mm Hg) zu erhalten. (DE-PS 31 44 705 C2).

Für alle Hämoglobin-Vernetzungsreaktionen - etwa mit Glutardialdehyd - gilt, daß die Vernetzungen sehr schwierig zu steuern sind und nur in grober Näherung reproduzierbare Vernetzungsprodukte erhalten werden können, wobei auch Ausbeuteverluste hingenommen werden müssen. Weiterhin ist die kolloidosmotische Aktivität dieser Polymerisate oft so klein, daß zur Korrektur des KOD weitere teure Additive, wie Albumin, zugesetzt werden müssen. (DE-PS 31 44 705 C2).

Da ferner die Viskosität der vernetzten Produkte gegenüber Hämoglobinlösungen gleicher Konzentration, bei gleichzeitig verringerter Wasserlöslichkeit, stark erhöht ist, versuchte man in der Folgezeit, definierte "Modifizierungsmittel" einzusetzen, die die Tetramerstruktur intramolekular durch Ausbildung kovalenter Bindungen vernetzen und gleichzeitig die Sauerstoffbindungsaffinität erniedrigen.

Benesch et al. beschrieben 1975 die Umsetzung von deoxygeniertem Hämoglobin mit 2-Nor-2-Formyl-pyridoxal-5-phosphat. Tatsächlich konnte nachgewiesen werden, daß das intramolekulare Vernetzungsprodukt im Tierexperiment nur sehr langsam ausgeschieden wird. Dies Produkt wies eine intravasale Halbwertszeit von T 1/2 = 24 Stunden bei einem Sauerstoff-Halbsättigungsdruck von P 50 = 3,46 kPa (26 mmHg) bis 3,72 kPa (28 mmHg) auf. Nachteilig ist die sehr umständliche Synthese dieses Modifizierungsmittels. In der US-PS 4 529 071 wird die intramolekulare Vernetzung in stromafreiem tetramerem deoxy-Hämoglobin mit Diaspirinestern und nachfolgender Modifikation mit Pyridoxylphosphat beschrieben. Es werden Produkte erhalten, die Halbsättigungsdrucke von 3,33 kPa (25 mmHg) bis 4,65 kPa (35 mmHg) aufweisen. Im Tierexperiment wurde am Kaninchen bei einer Dosis von 200 mg/kg eine intravasale Halbwertszeit um 20 Stunden beschrieben.

Nachteilig ist jedoch, daß mehrere Reaktionen mit Hämoglobin, nämlich die Umsetzung mit den Diaspirinestern, Pyridoxalphosphat und $NaBH_4$, notwendig sind.

In der US-PS 4 473 496 werden phosphorylierte, mit Perjodat oxidierte Ribosederivate, ausgehend von

Adenosin-5'-triphosphat oder von 5-Phosphoribosyl-1-pyrophosphat, beschrieben, die mit Hämoglobin intramolekular vernetzte Produkte ergeben. Die erhaltenen P 50-Werte liegen um 4,65 kPa (35 mmHg).

Aufgabe dieser Erfindung war es deshalb, durch eine einfache Reaktion mit nur einem einzigen Modifizierugsmittel neue Hämoglobinprodukte zur Verfügung zu stellen, deren P 50-Wert je nach Erfordernis in weiten Grenzen zwischen 2,66 kPa (20 mmHg) und 10,64 kPa (80 mmHg), bevorzugt zwischen 4,79 kPa (36 mmHg) und 9,33 kPa (70 mmHg) frei einstellbar ist.

Die Notwendigkeit, einen höheren $O_2$-Halbsättigungsdruck (P 50-Wert) als 3,59 kPa (27 mmHg) in den stromafreien Hb-Lösungen einzustellen, wird aufgrund der folgenden Überlegungen deutlich:

Ergebnisse von Modellrechnungen (Dissertation W. Gauch, Aachen, 1986) zeigen, daß die arteriovenöse Differenz der Sauerstoffsättigung und Sauerstoffkonzentration als Maß für die "Ausschöpfung" des oxygenierten Hämoglobins im Blut in Abhängigkeit des P 50-Wertes bei ansonsten gleichen Atmungsbedingungen ein Maximum durchläuft.

Ergebnis dieser Berechnungen ist, daß die Ausschöpfung des an Hämoglobin gebundenen Sauerstoffs in dem Bereich des P 50-Wertes von 5,32 kPa (40 mmHg) bis 7,98 kPa (60 mmHg) ein Maximum durchläuft. Es reicht deshalb nicht aus, den P 50-Wert eines Hämoglobin-haltigen Blutersatzmittels auf etwa 3,33 kPa (25 mmHg) bis 3,72 kPa (28 mmHg) einzustellen, sondern die Sauerstoffausschöpfung kann noch weiter maximiert werden, wenn der 50-wert in den genannten Bereich verschoben wird. Beispielsweise zeigt eine Modellrechnung, daß es möglich ist, eine Steigerung der arterio-venösen Sättigungsdifferenz um das 2,5fache zu erreichen, wenn der P 50-Wert in Erythrozyten von 3,59 kPa (27 mmHg) auf 8,25 kPa (62 mmHg) verändert ist. Unabhängig davon ist, ob in vivo weitere Kompensationsmechanismen (z.B. Veränderung des Herzzeitvolumens) wirksam werden, die etwa bei einem Blutverlust den verminderten Hämoglobingehalt ausgleichen können.

Ein weiteres Ziel der Erfindung ist es deshalb, ein Verfahren zu entwickeln, um den P 50-Wert von Hämoglobinlösungen bis in den Bereich der als "optimal" berechneten P 50-Werte, bei denen eine maximale Sauerstoffausschöpfung des Hämoglobins zu erwarten ist, verschieben zu können. Hämoglobinlösungen mit optimalen P 50-Werten lassen die folgenden Vorteile gegenüber den bestehenden Lösungsansätzen erwarten:

- Im Vergleich zu dem nicht optimalen P 50-Wert können geringere Volumina der Hb-Lösung an Patienten verabreicht werden, da die Sauerstoffausschöpfung des Präparates optimiert ist.
- Daraus folgt, daß eine wesentlich höhere Wirksamkeit, bezogen auf des eingesetzte Hämoglobin, erzielt werden kann.

Es ist bekannt, daß die Sauerstoffaffinität und damit der P 50-Wert von Hämoglobin A durch polymere Polyanionen, wie Heparin oder Dextransulfat, beeinflußt werden kann [P. Labrude et al., Hemoglobin Solutions as Oxygen Carriers: Ligands and Other Molecules Interactions with Hemoglobin in: Adv. Biosci. 1986, 54 (Oxygen Transp. Red Blood Cells), Seite 16 ff und Amiconi, G. et al., Eur. J. Biochem. 76, 339-343 (1977)].

Konzentrationsabhängig erhöht sich der P 50-Wert des Hämoglobins bei steigender Heparinkonzentration bis auf Werte von 7,98 (60 mmHg) bis 10,64 kPa (80 mmHg). In einer Publikation "Interaction Between Human Hemoglobin And Sulfated Polysaccharides: Indentification Of the Binding Site and Specificity" in: Affinity Chromatography and Biological Recognition, Ed. I. M. Chaiken, M. Wilchek, I. Parikh, Academic Press, 1983, wird festgestellt, daß

- in Mischungen von HbF, HbA, HbS und HbC unter den Bedingungen einer Gelektrophorese im Agar-Gel ein ungewöhnliches Trennverhalten des Hämoglobins auftritt. Dieser Effekt blieb aus, wenn der Agar gegen Agarose oder Agarose Gel mit geringem Sulfatgehalt ausgetauscht wird. Es wurde ferner in dieser Publikation festgestellt, daß das Gelationsverhalten von Sichelzellen-Hämoglobin (Hbs) durch Fucoidan, λ-Carragenan, Dermatansulfat und Heparin, nicht jedoch von Hyaluronsäure oder Chondroitin- 4- oder -6-sulfat gehemmt wird. Säulenchromatographisch wird ferner eine Interaktion von gereinigtem Hämoglobin mit λ-Carragenan nachgewiesen und die Assoziationskonstanten mit HbF, HbA und HbS ermittelt.

Es kann deshalb als bekannt angesehen werden, daß einige polyanionische Polymere wie Z.B. Heparin, Dextransulfat oder Dermatansulfat mit Hämoglobin in wäßriger Lösung Assoziate bilden, die konzentrationsabhängig den P 50-Wert und damit die Sauerstoffaffinität von Human-Hämoglobin(en) verringern.

Einige Versuche mit Heparin zeigen jedoch, daß diese Bindung von Heparin an Hämoglobin A zwar relativ fest ist, jedoch auch relativ hohe Heparinkonzentrationen gewählt werden müssen, um eine Verschiebung des P 50 in den Bereich von etwa 6,65 kPa (50 mmHg) zu erreichen.

So wird der P 50-Wert einer stromafreien Hämoglobinlösung mit 67 mg/ml HbA von 0,66 kPa (5 mmHg) auf 8,25 kPa (62 mmHg) verschoben, wenn 10 mg/ml Heparin zugemischt werden. Die Meßbedingungen zur Erfassung der Sauerstoffbindungskurve sind: 225 mM BISTRIS-Puffer, pH 7,4, 37 ° C.

Handelsübliches Heparin besteht aus einem Gemisch sulfatierter Glycosaminoglykanketten, die im Molekulargewichtsbereich von etwa 3 000 bis 30 000 liegen. Das Molekulargewichtsmaximum der Molekulargewichtsverteilung liegt bei etwa 12 000 bis 15 000.

Heparin ist ein Naturprodukt und wird hauptsächlich aus der Lunge und Darmschleimhaut (Mucosa) von Schlachttieren gewonnen. Im Handel am weitesten verbreitet sind Heparinsalze in der Natrium- oder Calcium-Form, die aus der Mucosa von Rindern und Schweinen und der Lunge von Rindern isoliert werden. Medizinisch wird Heparin als Antithrombotikum sowohl in der Prophylaxe von Thrombosen und Thrombo-Embolien als auch in therapeutischen Dosen zur Behandlung von thromboembolischen Komplikationen und in extrakorporalen Blutkreisläufen eingesetzt.

Die bedeutendste Nebenwirkung von Heparin besteht darin, daß insbesondere bei höheren Dosierungen durch die Blockade des plasmatischen Gerinnungssystems und Beeinflussung der Thrombozyten Blutungskomplikationen auftreten können.

Die biologische Aktivität des Heparins kann mit Hilfe von Gerinnungstests wie dem Test gemäß USP XX mit Schafsplasma oder der apTT (aktivierte Thromboplastinzeit) ermittelt werden. Die Aktivität wird in Internationalen Einheiten ausgedrückt. Als Standard für die o.g. Tests dient ein von der WHO etabliertes Vergleichs-Heparin, zur Zeit der 4. WHO-Heparinstandard. Ferner werden häufig auch chromogene Substrattests eingesetzt, die die Heparin-katalysierte Hemmung von Thrombin oder Faktor Xa als Testprinzip ausnutzen.

Die antikoagulatorische Heparinaktivität - gemessen mit der apTT oder dem Test gemäß USP XX - sinkt mit abnehmendem Molekulargewicht des Heparins. Die Aktivität handelsüblicher Heparine liegt um 150 E/mg.

Die chemische Heparinstruktur besteht weitgehend aus sich wiederholenden Sequenzen des dreifach sulfatierten Disaccharides $I_{2S}$-$A_{NS,6S}$ ($\alpha$-1,4-L-Iduronsäure-2-sulfat)-(D-Glucosamin N,6-disulfat). Dieser normalerweise in Heparin vorkommende Strukturbaustein kann durch mehrere Sequenzen unterbrochen oder ersetzt werden. B. Casu gibt in Nouv. Rev. Fr. Haematol., 26, 211-219 (1984) einen Überblick über durchschnittliche Heparinstruktur.

Heparine lassen sich ferner elektrophoretisch in basischen Puffern in Anwesenheit von mehrwertigen Kationen in zwei Fraktionen, die sogenannte fast-moving und die slow-moving-Komponente, auftrennen.

Ferner bestehen strukturelle Unterschiede, je nachdem, aus welchem Organ das Heparin gewonnen wurde. NMR-spektroskopisch ($^1H$ und $^{13}C$) lassen sich Typ A-(Mucosa) und Typ B-(Lungen) Heparine unterscheiden.

Insgesamt kann festgestellt werden, daß Heparin ein heterogenes Substanzgemisch darstellt, das sich beispielsweise hinsichtlich der Gesichtspunkte

- Provenienz (Tierspezies)
- Art des Salzes (z.B. Natrium oder Calcium)
- Molekulargewicht bzw. Molekulargewichtsverteilung
- fast/slow-moving-Anteile
- anionische Dichte
- Sulfatierungsgrad
- Gehalt von N-Acetyl-oder N-Sulfat-gruppen
- Typ A oder B
- der biologischen Aktivität, z.B. antikoagulatorische Aktivität
- ihrer Affinität zu Plasmaproteinen, wie etwa immobilisiertem AT III,

von anderen Glycosaminoglykanen unterscheiden läßt.

Wie bereits erwähnt, steigt der Einfluß von Heparin auf den P 50-Wert des Hämoglobins an, wenn Heparine mit geringerem Molekulargewicht als handelsübliches Heparin mit Hämoglobin in unterschiedlicher Konzentration unter definierten Bedingungen (pH, Temperatur, Ionenstärke, Puffer) vermischt werden.

Nun sind in der Literatur zahlreiche Verfahren bekannt, handelsübliches Heparin chemisch oder enzymatisch in Bruchstücke zu spalten oder durch Fraktionierung mit an sich bekannten Verfahren, wie Gelfiltration, fraktionierten Fällungsschritten mit organischen Lösungsmitteln oder Ultrafiltration, herzustellen bzw. zu isolieren.

Einen Überblick über die gängigen Verfahren gibt der bereits zitierte Artikel von Casu. Ferner sei auf die Publikation von B. Casu "Structure and Biological Activity of Heparin" in "Advanced Carbohydrate Chemistry and Biochemistry" verwiesen. Die genannten Trenn- und Spaltungsverfahren von Heparin und anderen sulfatierten Glycosaminoglykanen sind deshalb grundsätzlich bekannt.

Unter den aus der Literatur bekannten Spaltungsreaktionen von Heparin sind einige, insbesondere die Spaltung mit $HNO_2$ und $NaJO_4$, die zu Heparin bzw. Heparinfragmenten mit reaktiven Aldehydgruppen führen.

1. Salpetrige Säure spaltet die glycosidische Bindung zwischen dem Glucosamin-N-Sulfatrest und dem Uronsäurerest des Heparins, so daß am reduzierenden Ende des Heparinspaltproduktes reaktive 2,5-D-Anhydromannosereste entstehen. Salpetrige Säure reagiert mit den freien Aminogruppen der Glucosaminreste des Heparins, die bereits zu einem geringen Teil in handelsüblichem Heparin vorliegen (10 bis 50 µmol/g) oder unter den Reaktionsbedingungen im sauren pH-Wert-Bereich durch N-Deacetylierung oder N-Desulfatierung entstehen.

Salpetrige Säure kann durch sehr unterschiedliche Verfahren, die dem Fachmann bekannt sind, in situ erzeugt werden. Der einfachste Weg besteht darin, zu wäßrigen Heparinlösungen im sauren pH-Wert-Bereich Nitrit (z.B. Natriumnitrit) zuzusetzen und die Reaktion bei definierter Temperatur im Bereich von 2 bis 60 °C ablaufen zu lassen. Auch die Depolymerisation von Heparin mit $HNO_2$ in 1,2-Dimethoxyethan bei -20 °C sei hier beispielhaft als Herstellungsvariante genannt. Das Ausmaß der Spaltung wird durch die Bedingungen der Reaktion, insbesondere durch Verhältnis Heparin zu Nitrit, bestimmt. Um möglichst kleine Bruchstücke zu erhalten, wird mit einem hohen Überschuß an Nitrit gearbeitet. Der pH-Wert beeinflußt den Reaktionsablauf, indem zwar die Spaltungsreaktion rascher abläuft, jedoch im sauren pH-Wert-Bereich vermehrt mit Konkurrenzreaktionen wie N-Desulfatierung, O-Desulfatierung wie auch einer Hydrolyse der N-Acetylgruppen in dem Ausgangsheparin und den Reaktionsprodukten zu rechnen ist. Systematische Untersuchungen zeigten, daß im pH-Wert-Bereich von 2 bis 6 bevorzugt um pH 4,0 gearbeitet werden kann.

2. Weiterhin kann Heparin auch an der $C_2$ - $C_3$-Bindung nicht sulfatierter Uronsäurereste zunächst einer Perjodatoxidation unterworfen werden und nachfolgend die so erzeugten Dialdehyde mit Alkalihydroxidlösungen behandelt werden. Auch diese Spaltung liefert Fragmentierungsprodukte mit geringem Molekulargewicht, die gleichzeitig reaktive Aldehydgruppen tragen. Eine Vorschrift zur Oxidation von Heparin mit Perjodat wird gegeben in: B. Casu et al., Arzneim. Forschung/Drug Res. 36(I), Nr. 4 (1986), S. 637 ff.

3. Weiterhin können zunächst mit einem bekannten Verfahren, wie dem enzymatischen Abbau mit Heparinasen, oder einer Hydrazinolyse oder einem Fraktionierungsverfahren mittels Ultrafiltration, fraktionierter Fällung mit organischen organischen Lösungsmitteln oder Gelfiltrationen Heparinfraktionen mit geringen Molekulargewichten aus handelsüblichem Heparin hergestellt werden, und diese Spaltprodukte oder Fraktionen einem weiteren Abbau mit $HNO_2$ und/oder einer Perjodatoxidation mit $NaJO_4$ unterworfen werden.

4. Eine andere Möglichkeit, definierte Heparinfragmente mit reaktiven Aldehydgruppen zu erhalten, besteht darin, Heparin zunächst einer Spaltung mit $HNO_2$ gemäß 1 zu unterwerfen und in einem Folgeschritt gemäß 2 mit Natriumperjodat umzusetzen. Die Reihenfolge beider Schritte ist umkehrbar.

5. Ferner können Heparin oder beliebig anderweitig hergestellte Heparinspaltprodukte mit Aktivierungsreagenzien wie Bromcyan oder EDC [1-Ethyl-3-(3-dimethylaminopropyl)-carbodiimid] aktiviert werden. Solche Aktivierungsreagenzien für Polysaccharide und Proteine sind dem Fachmann aus der Affinitätschromatographie oder der Chemie zur Immobilisierung von Proteinen bekannt. Siehe "Methods in Enzymology" Band 44 und 34 und "Introduction to Affinity Chromatography", C.R. Low, North-Holland, 1979, Ed.: T.S. Work, E. Work.

So kann beispielsweise die Carboxylgruppe von Heparin auch mit N-Hydroxysuccinimid mit Hilfe von Dicyclocarbodiimiden aktiviert werden.

Die US-PS 4 496 550 beschreibt die Herstellung und Verwendung von Oligosacchariden (Heparinfragmenten), die durch Heparinspaltungsverfahren hergestellt sind:

a) Heparin wird mit $HNO_2$ in 1,

b) Heparin wird mit Nitrit in wäßriger Lösung umgesetzt.

c) Heparin wird mit Perjodat bei niedrigem pH-Wert und niedriger Temperatur behandelt.

d) Heparin wird enzymatisch mit Heparinasen behandelt.

e) Heparin wird depolymerisiert, indem zunächst die Carboxylgruppen verestert und darauf folgend einer alkalischen $\beta$-Elimination unterworfen werden.

f) Heparin wird teilweise durch partielle N-Desulfatierung depolymerisiert und ein Spaltungsschritt mit $HNO_2$ angeschlossen.

Weitere Verfahren der chemischen Spaltung unter Verwendung von $H_2O_2$/Ascorbat oder Persäuren (radikalische Depolymerisation) sind ebenfalls bekannt geworden. (Brit. Pat. Appl. 33615/77; US-PS 4 281 108; EP-A-012 067).

In "Carbohydrate Research" 138, 199-206 (1985) wird ferner eine Vorschrift zum erschöpfenden Abbau von Heparin aus Rinderlunge mit salpetriger Säure gegeben.

Der Erfindung liegt somit die Aufgabe zugrunde, bekannte Heparinfragmente mit Hämoglobin das als wässrige, stromafreie Lösung vorliegt, chemisch zu koppeln und auf diese Weise neue Sauerstoff-übertragende "Blutersatzmittel" herzustellen.

Gegenstand der Erfindung sind daher stromafreie Blutersatzmittel, die dadurch gekennzeichnet sind, daß man in an sich bekannter Weise hergestellte Fragmente von sulfatierten Glykosaminoglykanen, ausgewählt aus Heparin, Heparinoiden, Dermatansulfat, Heparansulfat, sulfatiertem Chitin und/oder Chitosan, wobei die Heparinfragmente Molekulargewichte von 1000 bis 10.000 aufweisen, mit Hämoglobin, welches aus verschiedenen Spezies, wie Mensch, Schwein, Rind, gewonnen werden kann, zu stabilen, kovalent miteinander verknüpften Addukten derart umsetzt, daß deren Sauerstoff-Bindungsverhalten durch die Einstellung des Verhältnisses von Hämoglobin zu Glycosaminoglykanfragment im Bereich von 2,66 kPa (20 mmHg) bis 10,66 kPa (80 mmHg) eingestellt wird, wobei das molare Verhältnis von Hämoglobin zu Heparinfragment im Bereich von 1 : 0,4 bis 1 : 5 eingestellt wird.

Das Sauerstoffbindungsverhalten wird vorzugsweise im Bereich von 4,79 kPa (36 mmHg) bis 9,33 kPa (70 mmHg), eingestellt.

Erfindungsgemäß wurde nun gefunden, daß sich Heparinfragmente mit Molekulargewichten von 1 000 bis 10 000, bevorzugt im Molekulargewichtsbereich 1 000 bis 3 000, die gleichzeitig reaktiv Gruppen, wie beispielsweise Aldehydfunktionen, tragen, mit Hämoglobin zu stabilen Produkten umsetzen lassen. Dabei entstehen Hämoglobin-Heparinfragment-Addukte, deren Sauerstoffbindungsverhalten, ausgedrückt durch den P 50-Wert, im Bereich von 2,66 kPa (20 mmHg) bis 10,66 kPa (80 mmHg) eingestellt werden kann.

Selbstverständlich können auch weitere sulfatierte Glycosaminoglykane, wie Dermatansulfat oder Heparansulfat, zunächst in Fragmente mit reaktiven Endgruppen gespalten werden und diese Fragmente mit Hämoglobin umgesetzt werden. Es ist ferner möglich, die zur Spaltung eingesetzten Rohstoffe zunächst einem Sulfatierungsschritt zu unterwerfen, um die Anzahl der O-Sulfatgruppen zu steigern. Verfahren zur Einführung von Sulfatgruppen als N- und O-sulfatester sind dem Fachmann bekannt. Es können Verfahrensvarianten von C.R. Ricketts, "Biochem. Journal" 51 129-133 (1952) oder O. Larm, et al., "Carbohydrate Research" 73, 332-336 (1979) angewandt werden.

In diesem Falle der zusätzlichen Einführung von Sulfatgruppen wird der Einfluß der Heparinspaltprodukte auf den P 50-Wert des Hämoglobins vergrößert, d.h. bereits mit geringeren Mengen Fragment können im Vergleich zu den nicht nach-sulfatierten Fragmenten größere Verschiebungen des P 50-Wertes erzielt werden. Es wurde ferner gefunden, daß auch sogenannte Heparinoide, d.h. chemisch mit Heparin verwandte Substanzen wie Pentosanpolysulfat SP 54$^R$ der Firma Bene-Chemie, München, oder Arteparon$^R$, der Firma Luitpold, München, ebenso wie Heparin durch Umsetzung mit $HNO_2$ und/oder Natriumperjodat aktiviert und mit Hämoglobin umgesetzt werden können.

Ferner sind Heparin-ähnliche sulfatierte Glycosaminoglykane, die aus Chitin bzw. Chitosan hergestellt werden können und eine Heparin-ähnliche biologische Aktivität zeigen, im Sinne der Erfindung ebenso wie Heparin als Ausgangsmaterialien für weitere Abbaureaktionen einsetzbar. Beispielsweise beschreiben Hirano, S. und Tanaka, T. in"Carbohydr. Res." 137, 205-215 (1985) "Effect of sulfated derivatives of chitosan on some blood coagulant factors" Chitosanderivate mit Heparinaktivität. Diese Produkte können ebenso dem weiteren Abbau bzw. einer Aktivierung mit $HNO_2$ zugeführt werden. Diese Abbauprodukte werden wieder mit Hämoglobin unter Bildung von Addukuten umgesetzt, deren P 50-Wert zu höheren Partialdrucken hin verschoben ist.

Im Zuge der Herstellung der Heparinfragmente müssen die zur Spaltung notwendigen Hilfsstoffe, insbesondere Nitrit, bis in den unteren ppm-Bereich (<0,1 ppm) abgetrennt werden, um unerwünschte Reaktionen (Oxidation zu Methämoglobin) zu vermeiden. Zur Charakterisierung der Heparinfragmente mit reaktiven (End-)Gruppen können folgende Daten herangezogen werden:

- Bestimmung des Gehaltes freier Aldehydgruppen
- Molekülmassenbestimmung
- Natriumgehalt
- optischer Drehwert
- Schwefelgehalt
- Osmolarität
- Spektrales Verhalten
- Restgehalt an Reagenzien, insbesondere Nitrit
- Gerinnungstests, z.B. gemäß USP XX

Insbesondere bei dem sehr einfach auszuführenden Heparinabbau mit $HNO_2$ (Natriumnitrit im sauren pH-Wert-Bereich) kann das Molverhältnis von Heparin/$HNO_2$ im Bereich von 1 : 0,2 bis zu 1 : 100 variiert werden. Siehe dazu auch: Knipmeyer, A.J.K., "Master Thesis", Twente University of Techno.ogy (1985).

Durch die zusätzliche Wahl von pH-Wert, Temperatur, Reaktionszeit ist es möglich, Heparinfragmente mit gewünschten Molekulargewichten und reproduzierbarem Aldehyd(Anhydromannose)-gehalt herzustellen.

Bei der näheren Untersuchung der Hämoglobinfragment-Addukte stellt sich überraschenderweise heraus, daß

- die intravasale Halbwertszeit der Hämoglobin-Heparinfragment-Addukte gegenüber nicht chemisch umgesetztem Hämoglobin, je nachdem, welches Heparin/Hb-Verhältnis gewählt wurde, stark verlängert ist;
- sich der P 50-Wert durch das Verhältnis Hb/Heparinfragment weitgehend einstellen läßt;
- sich stabile Addukte unter Ausbildung kovalenter Bindungen mit Hb ergeben;
- unter den gewählten Reaktionsbedingungen keine typischen hochpolymeren "Vernetzungsprodukte" entstehen;
- die Stabilität gegenüber Oxidation - bezogen auf die Bildung von Met-Hämoglobin - im Vergleich zu Hämoglobin vergrößert ist.
- Die Hämoglobin-Heparinfragment-Addukte können hergestellt werden, indem oxygenierte oder bevorzugt deoxygenierte Hämoglobinlösungen in einem Puffer im pH-Wert-Bereich 5,5 bis 8,5 in wäßriger Lösung mit den reaktiven Heparinfragmenten umgesetzt werden. In einem Folgeschritt wird der mögliche Überschuß der Heparinfragmente durch ein Ultrafiltrationsverfahren (Diafiltration) abgetrennt und gliechzeitig das Puffermilieu z.B. mit Tyrode- oder einem Dialyse-Puffer umgestellt.

Ein weiteres überraschendes Anwendungsgebiet von Heparinfragmenten (die gemäß den bekannten Verfahren hergestellt wurden) wurde zufälligerweise gefunden, indem nicht mit Hämoglobinlösungen, sondern mit intakten Erythrozyten bei Raumtemperatur in einem isotonischen BISTRIS-Puffer, pH 7,4, inkubiert wurde. Einfaches Mischen der Heparinfragmente in isotonischer Lösung mit Erythrozyten, wobei das molare Verhältnis von Hämoglobintetramer zu Heparinfragment von 1 : 0,5 bis zu 1 : 20 variiert wurde, ergab eine Erhöhung des P 50-Wertes der Erythrozyten bis zu 9,31 kPa (70 mmHg). Eine Hämolyse der Erythrozten konnte unter diesen Bedingungen nicht festgestellt werden. Auch nach Einschließen des gemäß Beispiel 1 hergestellten Heparinfragmentes mit DMSO nach der von Franco und Weiner gegebenen Vorschrift (US-PS 4 478 824) oder nach Inkorporierung des Heparinfragmentes in Liposomen und Inkubation mit Erythrozyten (EP-A-0 052 322) oder auch durch das von M. Chaissaigne et al. beschriebene Verfahren konnte stets eine Einschleusung des Heparinfragmentes in die Erythrozyten - gemessen durch die Erhöhung des P 50-Wertes - erreicht werden. Die erfindungsgemäß verwendeten Heparinfragmente eignen sich deshalb zur Einschleusung in Erythrozyten und erzielen dort eine Erhöhung des P 50-Wertes. Selbst nach vier Wochen Lagerung der so behandelten Erythrozytensuspensionen wurde noch ein gleichbleibend hoher P 50-Wert gemessen, so daß diese Heparinfragmente offensichtlich nicht in Erythrozyten abgebaut werden.

Selbstverständlich können die Hämoglobine mit erhöhtem P 50-Wert auch weiteren Folgeschritten, wie dem Einschluß in Hämosomen oder, wenn erforderlich, einer konventionellen Vernetzung mit üblichen bi- oder polyfunktionellen Vernetzungsmitteln wie Glutardialdehyd zugeführt werden.

Zwar sinkt- ähnlich wie bei pyridoxaliertem Hämoglobin - der P 50-Wert gegenüber dem unvernetzten Hämoglobin-Heparinfragment-Addukt ab, er liegt jedoch auch dann noch in dem als optimal berechneten Bereich des P 50-Wertes.

Die Heparinfragmente werden üblicherweise in wäßriger Lösung im pH-Wert-Bereich von 5,5 bis 8,5, bevorzugt bei 6,1 bis 7,8, mit Hämoglobinlösungen umgesetzt. Die Konzentration der Hämoglobinlösung liegt zwischen 0,5 und 40 %, bevorzugt bei 5 bis 9 Gew.-% Hämoglobintetramer. Geeignete Puffer sind beispielsweise Phosphatpuffer, Hydrogencarbonatpuffer oder BISTRIS-Puffer. Die Konzentration an puffernden Ionen liegt im Bereich 0,05 bis 1 M. Die Temperatur der Umsetzung kann im Bereich von 2° C bis zu 60° C gewählt werden, wobei die schonendste Umsetzung zwischen 10° C und 30° C erfolgen sollte. Die Möglichkeit, die Reaktionstemperatur bis zu 60° C erhöhen zu können, deutet bereits auf die hohe thermische Stabilität der Hämoglobin-Heparinfragment-Addukte hin. Üblicherweise und bevorzugt erfolgte jedoch die Modifizierungsreaktion mit den Heparinfragmenten bei Raumtemperatur.

Bevorzugt erfolgt die Modifizierungsreaktion unter Sauerstoffabschluß in Laborfermentern. Laborfermenter eignen sich insbesondere deshalb zur Umsetzung von Hämoglobin mit Heparinfragmenten, weil

- sie steriles Arbeiten ermöglichen;
- die apparative Möglichkeit zur Verfolgung von Temperatur, pH-Wert, $O_2$-Sättigung und Redoxpotential besteht;
- Rührer eingebaut werden können;
- die Zusammensetzung des Gasraums kontrolliert werden kann (Begasung mit Inertgas wie $N_2$);
- steriles Zuführen von Puffer, Reagenzlösung möglich ist;
- sterile Probennahme möglich ist;
- Temperierung möglich ist.

Die Verfahren zur Deoxygenierung von Hämoglobin-enthaltenden Lösungen sind dem Fachmann bekannt. Es wurden drei unterschiedliche Verfahrensvarianten ausgewählt:

a) Durchblasen von Stickstoff

b) Einblasen von Wasserstoff in Anwesenheit geeigneter Katalysatoren

c) Deoxygenierung mit Hilfe von mikroporösen Hohlfasern [R. Schmukler, Shu Chien; "Biorheology" 22, 21-29 (1985) "Rapid Deoxygenation of Red Cells and Hemoglobin Solution using Hollow Capillary Fibers"].

Zur Durchführung der Verfahrensvariante b) in der Hämoglobinlösung sind Edelmetallkatalysatoren wie beispielsweise Lewatit OC 1045 (Bayer AG, Leverkusen) suspendiert. Nach Einblasen von Wasserstoffgas wird der an Hämoglobin gebundene sowie der gelöste Sauerstoff gemäß

$$2H_2 + O_2 \rightleftharpoons 2H_2O$$

entfernt. Die Sauerstoffsättigung liegt in jedem Falle unter 0,05 % $O_2$. Nachdem der Hämoglobinlösung unter Kontrolle des $O_2$-Partialdruckes der Sauerstoff entzogen wurde, wird das Heparinfragment, gelöst in Puffer, steril zugetropft. Der Fortschritt der Modifizierungsreaktion kann zeitabhängig durch Auftragung einer Probe des Reaktionsgemisches über einen Kationenaustauscher (Mono S, Pharmacia) verfolgt werden. Üblicherweise ist eine Reaktionszeit von 5 Stunden bei Raumtemperatur ausreichend.

Das Verhältnis von Hämoglobin zu Heparinfragment entscheidet über die Eigenschaften (P 50-Wert) des Reaktionsproduktes. Als Bezugsbasis für Hämoglobin wird stets das Hämoglobintetramer mit Molekulargewicht 64 500 zugrunde gelegt. Die molaren Verhältnisse von Hb/Heparinfragment liegen üblicherweise zwischen 1 : 0,4 bis 1 : 5, bevorzugt bei etwa 1 : 3 bis 1 : 5. Da das Molekulargewicht des Heparinfragmentes nicht einheitlich ist, sondern wie Heparin eine Molekulargewichtsverteilung aufweist, kann nur mit mittleren Molekulargewichten des Fragmentes gerechnet werden (ca. 2000 D).

Zur Vereinfachung wurde deshalb ein Massenverhältnis von Heparinfragmenten/Hb von 1 : 7 bis 1 : 84 gewählt, wenn, Heparinfragmente mit Molekulargewicht 2 000 D eingesetzt werden. Eine andere Bezugsbasis bei $HNO_2$-gespaltenen Heparinfragmenten ist der Aldehydgehalt, der im photometrischen Test unter Verwendung von MBTH als Reagenz mit Formaldehyd als Standard bestimmt wird. Das gewählte Molverhältnis entspricht einem Verhältnis (Hb)/(Aldehydgehalt des Heparinfragmentes) von 1 : 0,4 bis 1 : 5 (bei einem mittleren Aldehydgehalt von 0,5 $\mu$mol/mg Heparinfragment).

In einigen Fällen wurde eine Reduktion mit Natriumborhydrid angeschlossen. Bezogen auf Hämoglobin wurde $NaBH_4$ in wäßriger Lösung in einem Molverhältnis von 1 : 20, bezogen auf Hämoglobin, im Überschuß zugetropft und bei Raumtemperatur bis zu 10 Stunden nachgerührt. In der Regel wurde dieser Schritt jedoch weggelassen, da die durch $HNO_2$-Abbau hergestellten Heparinfragmente stabil am Hämoglobin verknüpft sind.

In einer weiteren Verfahrensvariante wurde das Addukt aus Hämoglobin und Heparinfragment einer konventionellen Vernetzung mit Glutardialdehyd unterzogen. Auch diese Reaktion wird bevorzugt unter Sauerstoffausschluß durchgeführt.

Der Glutardialdehyd wurde bezogen auf Hämoglobin im Molverhältnis von Hb/Glutardialdehyd im Bereich 1 : 3 bis 1 : 7 als 10 %ige Lösung zugetropft. Der pH-Wert liegt im Bereich von 6,6. Nach einer Reaktionszeit von 1 Stunde bei 25 °C wurde die Vernetzungsreaktion durch Zugabe des zehnfachen molaren Überschusses (bezogen auf die zugegebene Konzentration Glutardialdehyd) mit Glycin gestoppt und nach einer Reaktionszeit von 3 Stunden wie üblich aufgearbeitet.

Zur Aufarbeitung des Reaktionsgemisches wurde zunächst die Hämoglobinkonzentration im Fermentergefäß auf 5 bis 7 % (entsprechend 50 bis 70 mg Hb-Tetramer pro ml Lösung) eingestellt und gegen Puffer, z.B. Tyrode's Puffer, diafiltriert.

Die zur Diafiltration verwendeten Ultrafiltrationsmembranen, beispielsweise MWCO 10000 D der Firma Millipore (Pellicon-System) wurden mit Transmembrandrucken bis zu 4 bar betrieben, um einen raschen Volumenaustausch erzielen zu können. Zur vollständigen Abtrennung der Reaktanten und Reagenzien von Hämoglobin wurde das Flüssigkeitsvolumen mindestens fünfmal ausgetauscht.

Üblicherweise wurde ein Tyrode's Puffer eingesetzt, der 0,8 g NaCl, 0,02 g KCl, 0,02 g $CaCl_2$, 0,01 g $MgCl_2 \cdot 6\,H_2O$, 0,005 g $NaH_2PO_4$, 0,1 g $NaHCO_3$, 0,1 g Glucose in 100 ml Lösung enthält. Der pH-Wert wurde, falls notwendig, auf pH 7,4 bei 37 °C mit NaOH bzw. HCl korrigiert.

Im Ultrafiltrat wurde die Konzentration des Heparinfragmentes unter Anwendung des photometrischen Tests mit Toluidinblau überprüft. Erstaunlicherweise lag die Kopplungsausbeute, bezogen auf die zur Reaktion eingesetzte Menge des Heparinfragments stets zwischen 96 und 99,5 %.

Die modifizierte Hämoglobinlösung wurde zur Sterilfiltration und Abtrennung der möglicherweise enthaltenen Pyrogene über eine 0,2 $\mu$m-Filtrationskerze mit positivem Zetapotential filtriert und in sterile Glasflaschen abgefüllt.

Zur Charakterisierung der erhaltenen Addukte aus Hämoglobin-Heparinfragment können folgende Analysenverfahren eingesetzt werden:

- Gerinnungstests (apTT): Diese Überprüfung gibt Hinweise auf die Anwesenheit von Stromabestandteilen oder gerinnungsaktiven Heparinfragmenten im Endprodukt.
- Bestimmung der Hämoglobinkonzentration.
- Ionenaustauschchromatographie über Mono S (Pharmacia).
- Bestimmung des Met-Hämoglobingehaltes.
- Bestimmung des Hämoglobin-Molekulargewichtes entweder mit einer analytischen Ultrazentrifugation oder routinemäßig durch Gelpermeationschromatographie.
- SDS-Gelelektrophorese [gemäß U.K. Laemmli, Nature 227, 680-685 (1970)].
- Bestimmung des kolloidosmotischen Druckes.
- Bestimmung der Osmolarität.
- Messung einer Sauerstoffbindungskurve unter Berechnung des P 50-Wertes und des Hillkoeffizienten.
- Messung der Viskosität.

Als Ergebnis der analytischen Überprüfung zeigte sich, daß weder in der SDS-Gelelektrophorese noch durch Gelpermeationschromatographie, beispielsweise über Superose$^R$ 12 (Pharmacia), eine Erhöhung des Hämoglobin-Molekulargewichtes gemessen werden konnte. Auch die Aminosäureanalytik eines sauren Hydrolysates des Reaktionsproduktes gab im Vergleich zu einem nicht-umgesetzten Hämoglobin keine Veränderung des Aminosäuremusters.

Eine Umsetzung kann jedoch eindeutig durch die folgenden Kriterien nachgewiesen werden:

- Ionenaustauschchromatographie über einen Kationenaustauscher Mono S (Pharmacia). Es ist mit steigendem Fortschritt der Reaktion eine weitere Signalgruppe im Elutionsprofil erkennbar, die gegenüber Hba bei kürzeren Retentionszeiten detektiert wird (siehe Abb. 5).
- Auftrennung der Hämoglobin-Heparinfragment-Addukte über RP-Chromatographie beispielsweise FPLC-Pharmacia; Typ Pro RPC HR 5/10. Diese Auftrennung zeigt eine Verschiebung der Retentionszeit der $\alpha$-und/oder $\beta$-Kette gegenüber dem nicht modifizierten Hämoglobin.
- Sauerstoffbindungskurve: der Hillkoeffizient sinkt bei hohen Umsetzungsgraden bis auf Werte um 1,0. Der P 50-Wert steigt, insbesondere bei einem Molverhältnis von Hämoglobin/Heparinfragment von 1 : 5 auf Werte um 7,99 kPa (60 mmHg).
- Unterwirft man das Reaktionsgemisch von Hämoglobin und Heparinfragment einer Ultrafiltration unter Verwendung einer nicht für Hämoglobin permeablen Membran, die jedoch für Heparinfragmente durchlässig ist, so kann im Ultrafiltrat freies Heparinfragment nachgewiesen werden. Nach Ablauf der Modifizierungsreaktion werden Heparinfragmente nur in Spuren im Ultrafiltrat nachgewiesen. Es kann deshalb eine Kopplungsausbeute von mehr als 96 %, bezogen auf die eingesetzte Heparinkonzentration, berechnet werden.

Unabhängig von diesen physiko-chemischen Kriterien lag die intravasale Halbwertszeit nach intravenöser Gabe des gemäß Beispiel 1 hergestellten sterilen, pyrogenfreien Hämoglobin-Heparinfragment-Addukts im Hund bei 10 Stunden. Im Vergleich dazu lag der Kontrollwert einer reinen, stromafreien, nicht modifizierten Hämoglobinlösung bei nur 2 Stunden. In beiden Fällen lag die Dosierung bei 750 mg Hämoglobin/kg KG.

Im Zuge weiterer Untersuchungen gezeigt werden, daß auch andere sulfatierte Glycosaminoglykanfragmente mit reaktiven Endgruppen zur Modifizierung von Hämoglobin ebenso wie die Heparinfragmente eingesetzt werden können. Beispielsweise wurde Chitosan zunächst im sauren pH-Wert-Bereich deacetyliert, mit bekannten Verfahren sulfatiert und wie Heparin zur Spaltung mit $HNO_2$ bei pH 4,0 umgesetzt. Auch bei dieser Reaktionssequenz konnten sulfatierte Glykanreste erhalten werden, die sich mit Hämoglobin umsetzen lassen und dabei den P 50-Wert erhöhen.

Die Hämoglobin-Heparinfragment-Addukte zeigen eine ungewöhnlich hohe thermische Stabilität. So gelang es beispielsweise, das gemäß Beispiel 1 hergestellte Präparat über 10 Stunden bei 60 °C zu erhitzen, ohne daß in Wasser unlösliche Präzipitate entstanden. Voraussetzung war, daß das Präparat in deoxygenierter Form vorlag. Dies erfolgte Hilfe einer der beiden beschriebenen Verfahrensschritte, Zuführung von Stickstoff und/oder Einleiten von Wasserstoff in Anwesenheit eines (Edelmetall)-Katalysators. Es konnte ferner gezeigt werden, daß die Heparinfragment-Hämoglobin-Addukte unter Zusatz von Gerüstbildnern wie Glycin, Sorbit und Glucose, lyophilisiert werden können. Bei diesem Prozeßschritt stieg der Met-Hämoglobingehalt bis auf Werte um 4,5 %.

Einfluß der Variation des P 50-Wertes auf verschiedene Parameter des $O_2$-Transportes

Mit Hilfe eines Computerprogrammes, das mit dem beschriebenen Gerät zur Bestimmung von Sauerstoffbindungskurven [siehe Gersonde (1985) s.o., W. Gauch, Dissertation RWTH Aachen 1986] kombiniert

wurde, ist es möglich, auf Grundlage der gemessenen P 50-Werte und unter Annahme der Parameter $P_a$, $O_2$ = 12,63 kPa (95 mmHg), $p_v$, $O_2$ = 5,32 kPa (40 mmHg), einem Herzminutenvolumen von 4700 ml/Minute, einer Hb-Konzentration von 6,7 %, einem Totraumatemzeitvolumen (VT) von 5600 ml/Minute, einem Totraumvolumen (VD) von 10 % und einem $O_2$-Gehalt von 21 % $O_2$-Transportparameter wie die arteriovenöse Differenz, $O_2$-Angebot und $O_2$-Verbrauch zu simulieren.

Tabelle 1 zeigt den Vorteil des erhöhten P 50 eines modifizierten Hämoglobins gegenüber der unmodifizierten Hämoglobinlösung: die gefundene erhöhte arteriovenöse Konzentrationsdifferenz bedeutet eine vermehrte Ausschöpfung des an Hämoglobin gebundenen Sauerstoffs um den Faktor 2,1 (s. Tabelle 1).

**Tabelle 1**

**Arteriovenöse Differenz $avDO_2$ bei normaler und rechtsverschobener $O_2$-Bindungskurve**

| P 50 kPa (mmHg) | $P_a,O_2$ kPa (mmHg) | $P_v,O_2$ kPa (mmHg) | $C_a,O_2$ ($mlO_2$/ml) | $C_v,O_2$ ($mlO_2$/ml) | $avDO_2$ ($mlO_2$/ml) |
|---|---|---|---|---|---|
| 1,51 (11,39) | 12,63 (95) | 5,32 (40) | 0,0880 | 0,0772 | 0,0107 |
| 7,07 (53,16) | 12,63 (95) | 5,32 (40) | 0,0618 | 0,0393 | 0,0225 |

($P_a,O_2$ = arterieller Sauerstoff-Partialdruck; $P_v,O_2$ = venöser Sauerstoff-Partialdruck; $C_a,O_2$ = arterielle $O_2$-Konzentration; $C_v,O_2$ = venöse $O_2$-Konzentration; $AvDO_2$ = arteriovenöse Sauerstoff-Konzentrationsdifferenz

Die im Folgende erwähnten Abbildungen haben die nachstehende Bedeutung

| | |
|---|---|
| Abb. 1: | Sauerstoffbindungskurven Hämoglobin-enthaltender Lösungen |
| Abb. 2: | Elutionsprofil des gemäß 1.1 hergestellten Heparinfragmentes |
| Abb. 3: | Elutionsprofil eines handelsüblichen Heparin-Natriumsalzes |
| Abb. 4: | SDS-Polyacrylamidgelelektrophorese |
| Abb. 5: | Ionenaustauschchromatographie über FPLC-Mono S |
| Abb. 6: | FPLC-Reversed-phase-Chromatographie |
| Abb. 7: | $O_2$-Bindungskurven |
| Abb. 8 A: | UV-VIS-Spektrum (Hämoglobin + Inositol-Hexaphosphat) |
| Abb. 8 B: | UV-VIS-Spektrum (Hämoglobin + Heparinfragment) |
| Abb. 9 A: | IR-Spektrum eines unfraktionierten Heparins |
| Abb. 9 B: | $^1$H-NMR-Spektrum eines unfraktionierten Heparins |
| Abb. 9 C: | $^{13}$C-NMR-Spektrum eines unfraktionierten Heparins |
| Abb. 10 A: | IR-Spektrum eines gemäß Beispiel 1 hergestellten Heparinfragmentes |
| Abb. 10 B: | $^1$H-NMR-Spetrum gemäß Beispiel 1 hergestellten Heparinfragmentes |
| Abb. 10 C: | $^{13}$C-NMR-Spektrum eines gemäß Beispiel 1 hergestellten Heparinfragmentes |
| Abb. 11 A: | IR-Spektrum eines gemäß Beispiel 2 hergestellten Heparinfragmentes |
| Abb. 11 B: | $^1$H-NMR-Spektrum eines gemäß Beispiel 2 hergestellten Heparinfragmentes |
| Abb. 11 C: | $^{13}$C-NMR-Spektrum eines gemäß Beispiel 2 hergerstellten Heparinfragmentes |
| Abb. 12 A: | IR-Spektrum eines gemäß Beispiel 3 hergestellten Heparinfragments |
| Abb. 12 B: | $^1$H-NMR-Spektrum eines gemäß Beispiel 3 hergestellten Heparinfragmentes |

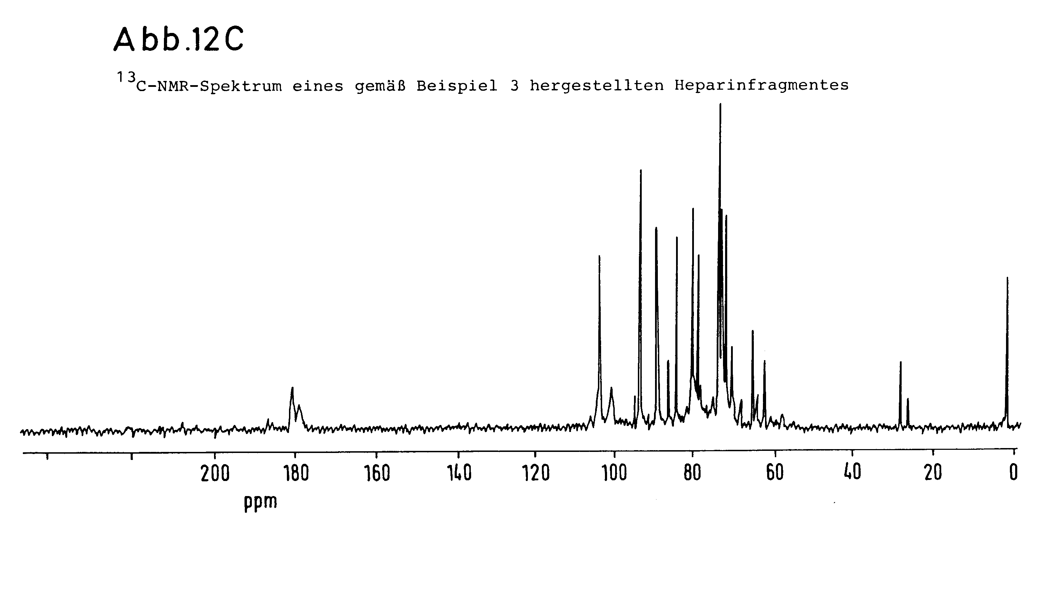

Abb. 12 C: $^{13}C$-NMR-Spektrum eines gemäß Beispiel 3 hergestellten Heparinfragmentes.

Einfluß der Variation des Hämoglobin/Heparinfragment-Verhältnisses auf den P 50-Wert

Wie die Beispiele 1 bis 7 zeigen, sind Fragmente des Heparins oder Heparin-ähnlicher Ausgangsmaterialien verschiedenen Ursprungs geeignet, den P 50-Wert nach kovalenter Kopplung mit Hb zu erhöhen.

Der Grad der Rechtsverschiebung der $O_2$-Dissoziationskurve des Hämoglobins ist bei konstantem Molekulargewicht und Aldehydgehalt eines Heparinfragmentes auch von dem Verhältnis Hb/Heparinfragment abhängig.

Die folgenden Untersuchungen - mit zwei verschiedenen Heparinfragmenten durchgeführt - belegen, daß allein durch Einstellung des Verhältnisses Hb/Heparinfragment der P 50-Wert in einem weiten Bereich variierbar ist:

In separaten Ansätzen wurde je 1 ml deoxygenierten Hämoglobin-Lysates (6,7 % Hba-Konzentration) mit steigenden Mengen an Heparinfragment inkubiert (4 Stunden, Raumtemperatur), das Hämoglobin-Heparinfragment-Addukt durch Chromatographie über eine Sephadex G 75-Säule, die mit BISTRIS-Puffer (pH 7,4) äquilibriert wurde, von freien Heparinfragmentresten abgetrennt und entsalzt, mit Ultrafiltration auf 6 % Hämoglobingehalt ankonzentriert und der P 50-Wert bei 37° C ermittelt.

Die folgende Tabelle zeigt die Ergebnisse:

| Heparinfragment (mg/ml Hb-Lösung) | P 50 (kPa/mmHg) (Fragment Bsp.1) | P 50 (kPa/mmHg) (Fragment Bsp.3) |
|---|---|---|
| 0,2 | 1,61 (12,1) | 1,50 (11,3) |
| 0,8 | 4,28 (32,2) | 2,87 (21,6) |
| 2,0 | 4,20 (31,6) | 3,27 (24,6) |
| 3,0 | 4,92 (37,0) | 4,31 (32,4) |
| 4,0 | 5,36 (40,3) | 5,08 (38,2) |
| 10,0 | 8,291 (62,34) | 9,31 (70,0) |

UV/VIS-Spektroskopische Dokumentation des Einflusses von Heparin-Fragment auf die Hämoglobin-Struktur

Um zu überprüfen, ob ein Heparinfragment bereits bei Mischung mit Hämoglobinlösungen möglicherweise Veränderungen der Hämoglobinstruktur bewirkt, die sich als Veränderungen des spektralen Verhaltens im UV/VIS-Spektrum im Vergleich zu einer Kontrolle ohne Heparinfragment nachweisen lassen, wurde das folgende Experiment durchgeführt. Inositolhexaphosphat, das ionisch an Hämoglobin mit hoher Bindungskonstante bindet und den P 50-Wert bei Mischung mit Hämoglobin zu hohen Sauerstoffpartialdrucken verschiebt, wurde als (Positiv-)Kontrolle mitgeführt.

Hämoglobinlösungen in Puffer (50 mM Natriumphosphat, pH 6,8) werden in steigenden Mengen mit Heparinfragment und in separaten Experimenten mit Inositolhexaphosphat vermischt und im Spektral-Bereich von 190 bis 700 nm registriert. Die Hämoglobinkonzentration lag stets bei 0,1 mg/ml. Die gewählten Molverhältnisse für Hb/Heparinfragment bzw. Hb/Inositolhexaphosphat liegen bei 1 : 1, 1 : 3, 1 : 5, 1 : 10, 1 : 20. Als Referenz wurde Hämolgobinlösung (0,1 mg/ml) verwendet. Abb. 8A und 8B zeigen das Ergebnis der Messungen.

Als Ergebnis bleibt festzustellen, daß bei 400 nm sowohl mit Heparinfragment wie auch mit Inositolhexaphosphat konzentrationsabhängig eine intensive Verminderung der Extinktion zu beobachten ist. Das UV/VIS-Spektrum von Heparin bzw. Inositolhexaphosphat weist im Bereich von 400 nm keine Absorption auf.

BEISPIELE

Beispiel 1

1.1. Herstellung eines niedermolekularen Heparinfragmentes durch Abbau mit $HNO_2$ aus Heparin-Natrium

50 g handelsübliches Heparin-Natriumsalz aus Schweinemucosa werden in einem Liter 0,05 M Natriumacetatpuffer pH, 4,0 gelöst. Die Fragmentierungsreaktion wird durch Zugabe von 400 ml einer 4 Gew.-%igen Natriumnitritlösung gestartet und bei 25°C 24 Stunden lang unter dem Abzug gehalten. Darauf wird mit 5 %iger NaOH auf pH 7,0 neutralisiert. Das Molverhältnis von Heparin (MW = 13 000) und Natriumnitrit liegt bei dieser Heparinspaltung bei 1 : 67.

Zur weiteren Aufarbeitung wird im Cuprophan-Dialyseschlauch gegen Wasser solange dialysiert, bis im Dialysat keine Nitritionen mehr nachweisbar sind. Zum Test des Nitrits wird das "Merck-Aquaquant"-System eingesetzt. Gleichzeitig wird überprüft, ob und wieviel Heparinfragment im Dialysat durch die Dialysemembran durchritt. Die Dialyse wird unterbrochen, wenn kein Nitrit im Dialysat mehr nachweisbar ist.

Für größere Ansätze wird mit Hilfe einer Millipore-Ultrafiltrationsmembran mit einem Cut-off von 1 000 D diafiltriert. Nach Dialyse oder Diafiltration des Reaktionsproduktes wird entweder lyophilisiert oder die Festsubstanz durch Sprühtrocknung isoliert. Die Ausbeute liegt bei ca. 70 %.

Die Heparinkonzentration im Dialysat oder Ultrafiltrat wird ermittelt, indem 100 µl einer Probelösung mit 2,5 ml einer Reagenzlösung (20,0 mg Toluidinblau/1000 ml) vermischt werden und die Extinktion bei 623 nm gegen den Reagenzienblindwert gemessen wird. Die Auswertung erfolgt durch Vergleich mit einer gleichartig erstellten Eichkurve. Dieses photometrische Verfahren ist beschrieben in: Paday, J.F. "Chemistry and Molecular Biology of the Intercellular Matrix" Vol. 2, Ed.: E.A. Ballazs, Academic Press 1970, Seite 1007-1031 und Silbert, Biochem. Biophys. Res. Commun. 69 S.70 ff (1976).

1.2 Charakterisierung des Heparinfragmentes

Die Bestimmung des Aldehydgehaltes des Fragmentes ergab: 0,52 µmol/mg.

Methode:

Der Aldehydgehalt wird nach der von B. Kakac und Z.J. Vejdelek beschriebenen Methode in "Handbuch der photometrischen Analyse organischer Verbindungen", Band 1, S. 233, Verlag Chemie, mit MBTH (3-Methyl-2-benzothiazolinonhydrazon-Hydrochlorid) bestimmt. Als Eichstandard werden Formaldehydlösungen eingesetzt.

- Optischer Drehwert: + 45,5°
- Osmolarität einer 5 %igen Lösung: 195 m Osm
- Spektren $^{13}C$, $^{1}H$ im Vergleich zu handelsüblichem Heparin (s. Abb. 9 bis 10)
- Gerinnungsaktivität: 0,6 E/mg (USP XX)
- Molmassenverteilung: 1 500 D bis 2 500 D
- Mittleres Molekulargewicht: 2 000 D.

Die Bestimmung der Molmassenverteilung erfolgt mittels Gelpermeationschromatographie über die Säulen Bondagel E 125 und E linear (Fa. Waters Königstein).
HPLC-Gerät: HP 1084 (Hewlett-Packard)
Probenkonzentration 1 mg/ml
Probenvolumen: 10 ul
Eluent: 0,1 M $Na_2HPO_4$ (pH 5,0)/$H_2O$ = (30 : 70)
Flow: 0,5 ml/Minute
Detektor: UV, Meßwellenlänge: 205 nm.

Abb. 2 zeigte ein Elutionsdiagramm der GPC-Trennung im Vergleich zu dem nicht gespaltenen handelsüblichen Heparin (ABB. 3). Das Molekulargewicht des Peakmaximums liegt bei 2 000, das des ungespaltenen Heparins bei 13 000. Das Molekulargewicht wird einer Eichkurve entnommen, die mit Hilfe von Heparinfraktionen bekannter Molekulargewichte erstellt wurde.

Beachtenswert ist die sehr geringe antikoagulatorische Aktivität des Heparinfragmentes, die bei 0,6 E/mg, bezogen auf den 4. WHO-Standard, liegt.

1.3 Herstellung einer stromafreien Hämoglobinlösung

Ausgehend von 15 Beuteln (500 ml) Human-Erythrozytenkonzentrat (Blutbank) wurden nach Tieffrieren bei -20°C und Wiederauftauen ca. 5 Liter Lysat erhalten, die mit 15 Litern Aqua a.i. verdünnt werden.

Der pH-Wert der Lösung wurde mit 0,1 N HCl (3 Liter) auf 5,5 eingestellt, und mit Auqua a.i. auf ein Gesamtvolumen von 25 Litern aufgefüllt.

Die Durchführung aller Filter- und Diafiltrationsschritte erfolgte ab diesem Schritt bei 4 ° C. Zur Sedimentation der Stromabestandteile wurde die Lösung zur Abtrennung der Stromareste 24 Stunden bei 4 ° C stehengelassen.

Es schloß sich eine Filtration über Sartopure 0,2 µm-Filterkerzen an. Der pH-Wert des Filtrates wurde anschließend mit 0,1 N NaOH (ca. 4 Liter) auf 6,8 eingestellt. Unter Einsatz des Pellicon-Kassettensystems (Millipore; MWCO: 10 000 D) wurde auf 10 Liter Gesamtvolumen eingeengt.

Der pH-Wert wurde nun auf 6,8 eingestellt, woraufhin die Lösung über einen 0,2 µm-Filter in ein 12-Liter-Fermentergefäß filtriert wurde (Hb-Konz.: 8,7 %; Volumen: 10 Liter).

Bei Raumtemperatur wurde steriler Stickstoff eingeblasen, bis ein Sauerstoffrestgehalt von höchstens 0,05 % in der Lösung erreicht wurde (die bei dieser Temperatur gemessene $O_2$-Löslichkeit in Wasser wurde als 100 % gesetzt. Der Met-Hb-Gehalt betrug 1,4 %.

Nun wurden ca. 5 Liter als Kontroll-Lösung abgezweigt und einer Diafiltration gegen 25 Liter einer mit $N_2$ gespülten Tyrode's-Pufferlösung unterworfen ("stromafreies Hb-Lysat").

Die restlichen 5 Liter des unbehandelten Lysates (Hb-Konzentration 87 mg/ml) werden mit dem Heparinfragment gemäß 1.1 (34 g gelöst in 100 ml aqua a.i.) versetzt. Unter diesen Bedingungen wird ein Molverhältnis von Hb/Heparinfragment von 1 : 2,5 erreicht. Unter Rühren und Stickstoffbegasung wird das Reaktionsgemisch über 4 Stunden bei Raumtemperatur belassen. Auch hier wurde anschließend gegen 25 Liter einer mit $N_2$ gespülten Tyrode's-Puffer-Lösung diafiltriert, über 0,2 µm-Filter filtriert und diese Lösung in sterile Flaschen abgefüllt. Diese Lösung wird Hämoglobin-Präparat genannt.

Zusammensetzung des Tyrode's-Puffer:

0,8 g NaCl, 0,02 g KCl; 0,02 g $CaCl_2$, 0,005 g $NaH_2PO_4$, 0,01 g $MgCl_2 \cdot 6H_2O$; 0,1 g $NaHCO_3$; 0,1 g Glucose in 100 ml (pH 7,48).

Charakterisierung des Produktes gemäß 1.3

a) SDS-Polyacrylamidelektrophorese

Die Auftrennung wurde gemäß der von Laemmli, U.K. (1980) ("Cleavage of Structural Proteins During the Assembly of the Head of Bacteriphage T4", Nature 227, 680-685) publizierten Methode durchgeführt.

Die Auftrennung des Hämoglobin-Proteinkomplexes erfolgt unter denaturierenden Bedingungen, so daß - wie in Abb. 4 dargestellt - insbesondere monomere Protein-Hämoglobinuntereinheiten ($\alpha$-Globin bzw. $\beta$-Globin: M.G. 16 400 D bzw. 15 850 D) im Gelmuster erscheinen.

Wie oben erwähnt zeigen sich in bezug auf das Molekulargewicht zwischen dem Kontrol-Lysat und der modifizierten Probe mit diesem Trennsystem keine Unterschiede. Anhand der nachfolgend beschriebenen Methode wurde jedoch eine geringfügige Erhöhung des Molekulargewichtes gefunden.

b) Molekulargewichtsbestimmung mittels analytischer Ultrazentrifugation

Mit Hilfe der analytischen Ultrazentrifugation (Absolutmethode) wurde das Molekulargewicht des Hämoglobin/Heparin-Adduktes mit 68 800 D gegenüber dem nativen Heparin mit 64 500 D bestimmt.

c) Inonenaustauschchromatographie über FPLC-Mono S

Das gebundene Heparinfragment verändert die Gesamtladung des Hämoglobin-Tetramers stark. Eine Ionenaustauschchromatographie zeigt eine Veränderung des Elutionsprofils der mit Heparinfragment modifizierten Hämoglobinlösung im Vergleich zu unbehandeltem Hämoglobin, wie Chromatographie über einen Kationenaustauscher (Mono S, Pharmacia) ergab.

Methode:

"Determination of $HBA_{1c}$ using a monodisperse cation exchanger", 5th European Congress of Clinical Chemistry, Budapest, Hungary, July 1983, Jeppsson, J.-O., Englund, H., Nylund, V. et al.

Die Auftrennung erfolgt über einen Kationenaustauscher (Mono S Type HR 5/5, Pharmacia Fine Chemicals, Uppsala) mittels einer Gradientenelution.

(Puffer A: 0,01 M Malonsäure pH 5,7;
Puffer B: 0,01 M Malonsäure pH 5,7 mit 0,3 M LiCl).

Detektion bei 405 nm.

Die Abb. 5 zeigt entsprechende Chromatogramme für ein mit Heparin-Effektor behandeltes Hb-Präparat (a) und ein natives, unbehandeltes Hb-Lysat (b). Der angelegte Salzgradient (% Anteil Puffer B) ist ebenfalls eingezeichnet.

Der Effekt des zugesetzten Heparinderivates wird durch die Verschiebung der im Chromatogramm später eluierenden Peakgruppe (in Abb. 5 mit II bezeichnet) zu einer schon bei niedriger Ionenstärke eluierenden Peakgruppe (mit I bezeichnet) verdeutlicht. Die Umsatzrate ist durch das Integralverhältnis beider Peakgruppen quantifizierbar (Peak I/Peak II = 72/28). Dieses Verhältnis wurde im Falle des Kontroll-Lysates mit 12/88 bestimmt. Daraus läßt sich eine Umsatzrate von 68,2 % berechnen.

d) "Reversed-phase"-Chromatographie über FPLC

Es erfolgt eine Auftrennung des Hämoglobinmoleküls in seine $\alpha$- bzw. $\beta$-Untereinheiten. Die durch Umsetzung mit dem Heparinfragment hervorgerufene Modifizierung des Hämoglobins bzw. seiner Untereinheiten wird dadurch belegt, daß das in Abb. 6A mit I bezeichnete Signal ($\beta$-Kette) in der modifizierten Hämoglobin-Lösung Abb. 6B nicht mehr sichtbar ist. Demgegenüber wird ein neues Signal III in Abb. 6B einer höheren Retentionszeit eluiert, das in Abb. 6A fehlt.

Methode:

"An integrated approach to the analysis of human hemoglobin variants by combining IEF, FPLC and titration curve analysis"; Electrophoresis '83, Walter de Gruyter & Co., Berlin, 1983, Wahlström, L., Nylund, V., Burdett, P. et al.

Die eingesetzte Säule (Pharmacia Fine Chemicals AB, Uppsala) "Pro RPC HR 5/10" wurde mit einem Gradienten aus 39 % Acetonitril 0,3 % Trifluoressigsäure (A) und 45 % Acetonitril, 0,15 % Trifluoressigsäure (B) betrieben (Detektion erfolgt durch Messung der Extinktion bei 280 nm; Flußrate: 0,2 ml/min).

e) Sauerstoffbindungskurve ($O_2$-Affinität)

Mit dem von Sick, H. und Gersonde, K. (1985) ("Continuous Gas-Depletion Technique for Measuring $O_2$-Dissociation Curves of High-Affinity Hemoglobin" Anal. Biochem. 146, 277-280) entwickelten Verfahren wurde das Sauerstoffbindungsverhalten des modifzierten Hb-Präparates geprüft. In der Abb. 7 sind diese Ergebnisse den Daten, die man mit einem unmodifizierten HB-Lysat erhält, gegenübergestellt:
Für das unbehandelte Hb-Lysat (Abb. 7a, Vergleichsbeispiel 1) ergibt sich ein P 50 von 0,854 kPa und ein Hill-Koeffizient (N) von 2,25, für das modifizierte Hb-Präparat (Herstellung gemäß 1.3, Abb. 7b ein P 50 von 8,367 kPa und ein Hill-Koeffizient von 1.07.

f) Viskosität:

Mit einem Kapillarviskosimeter nach Ubbelohde der Fa. Schott/Mainz wurde die Viskosität der modifizierten Hämoglobinlösung mit 1,45 cSt bestimmt ($H_2O$: 0,73 cSt; Vollblut: 2,45 cSt; Messung bei 37 °C).

Vergleichsbeispiel 1

5 Liter der gemäß Beispiel 1.3 hergestellten Kontroll-Lösung wurden abgezweigt und einer Diafiltration gegen 25 Liter einer mit $N_2$ gespülten Tyrode's-Pufferlösung unterworfen.

Nach einer Steril- und Pyrogenfiltration über ein 0,2 $\mu$m-Filter wird die Lösung direkt steril in 500 ml Flaschen abgefüllt. Diese Lösung wird auch als stromafreies Hb-Lysat bezeichnet.

Das Ergebnis der analytischen Untersuchungen der Lösungen des Beispiels 1 und des Vergleichsbeispiels 1 ist in der folgenden Tabelle dargestellt:

| | Hämoglobin-Präparat Beispiel 1 | stromafreies Hb-Lysat, Vergleichsbsp. 1 |
|---|---|---|
| HbA | 9,3 % | 7,6 % |
| Met Hb | 3,6 % | 3,4 % |
| Pyrogentest | Endotoxinfrei | Endotoxinfrei |
| P 50 | 8,376 kPa (62,98 mmHg) | 0,854 kPa (6,42 mmHg) |
| pH-Wert (37°C) | 7,4 | 7,4 |
| Osmolarität | 277 m Osmol | 270 m Osmol |
| Phospholipidanteile (Stroma) | nicht nachweisbar untere Nachweisgrenze: 1 µg/ml | nicht nachweisbar untere Nachweisgrenze: 1 µg/ml |
| K.O.D. | 4,51 kPa (33,9 mmHg) | 3,95 kPa (29,7 mmHg) |
| Viskosität (Kapillarviskosimeter nach Ubbelohde) | 1,45 cSt | 0,96 cSt |

## Beispiel 2

Herstellung eines Heparinfragmentes aus Mucosa-Heparin durch $NaJO_4$-Behandlung

2 g handelsübliches Natrium-Heparin (Mucosa) mit einem Molekulargewicht 13.500 werden in 40 ml eines 50 mM Natrium-Acetat-Puffers (pH 4,0; Einstellung mit Essigsäure gelöst.
Anschließend werden 140 mg $NaJO_4$ zugegeben; es folgt eine Inkubation über 6 h bei 2 bei 4°C unter Lichtausschluss. Das Molverhältnis von Heparin/$NaJO_4$ liegt bei 1/4,2.

Danach wird der pH-Wert mit 3N NaOH auf 12,0 gebracht und nach 30 min wieder auf 7,0 zurückgestellt (4N HCl). Es erfolgt eine Dialyse in Cuprophan-Dialyseschläuchen (Cut-off = 1.000 D) bis im Dialysat keine Chlorid-Ionen mehr nachzuweisen sind (Titration mit 0,1 N $AgNO_3$). Abschließend wird lyophilisiert oder sprühgetrocknet.

Für das nach dieser Variante hergestellte Heparinfragment wurden folgende analytische Daten gefunden:
Molekulargewicht: 2.400 D
Aldehydgehalt: 0,45 µmol/mg.

1000 ml einer Hämoglobinlösung (64 mg/ml HbA) werden zunächst unter Stickstoff - wie in Beispiel 1 - deoxygeniert und bei Raumtemperatur mit 10 g des mit Perjodat umgesetzten Heparinfragmentes während 3 h inkubiert. Das Molverhältnis von Hämoglobin zu Heparinfragment liegt bei 1 : 5.

Eine Probe (0,5 ml) der Reaktionsmischung wird über eine mit BISTRIS-Puffer äquilibrierte Säule (Sephadex G 75, Pharmacia Fine Chemicals, Uppsala; 1 cm × 18 cm; Durchflußgeschwindigkeit: 1 ml/30 min) entsalzt. Kontrolluntersuchungen zeigten, daß Hämoglobin unter diesen Bedingungen von möglicherweise noch in der Reaktionsmischung verbliebenem, nicht mit Hb umgesetztem Heparinfragment abgetrennt wird. Die entsalzte Hämoglobinlösung wird durch Ultrafiltration auf einen Hämoglobingehalt von 7,3 %

ankonzentriert und die Sauerstoffbindungskurve gemessen. Es wurde ein P 50-Wert von 7,33 kPa (55 mmHg) ermittelt.

Die Chromatographie über Mono S-Ionenaustauscher zeigte ein Peakgruppenverhältnis von 65, 4/34,6 (vgl. Abb. 5) und damit eine Umsatzrate von 60,68 % (natives Hb-Lysat zeigt im Chromatogramm der Mono S-FPLC ein Peakgruppen-Verhältnis von 12/88).

Beispiel 3

Herstellung eines Heparinfragmentes aus Mucosa-Heparin durch ein kombiniertes Abbauverfahren mit $NaNO_2/NaJO_4$

3.1.

10 g Natrium-Heparin (Mucosa) wurden in 200 ml einer 50 mM Natriumacetat-Lösung (pH 4,0; mit Essigsäure einstellen) gelöst und mit 80 ml einer 4%igen $NaNO_2$-Lösung versetzt.

Es wird 3,5 h bei Raumtemperatur gerührt; anschließend werden 5 g (= 25 mmol) $NaJO_4$ zugefügt, und es folgt eine Inkubation für 48 h bei 2 - 4 °C.

Abschließend wird dialysiert oder diafiltriert bis im Dialysat bzw. Diafiltrat keine $JO_4^{\ominus}$ - bzw. $NO_2^{\ominus}$ - Ionen mehr nachzuweisen sind.

(Nachweis der $NO_2^{\ominus}$ -Ionen mit "Aquaquant" (Merck AG). Das feste Reaktionsprodukt wird durch Lyophilisation gewonnen.

Für das Heparinfragment wurden die folgenden analytischen Daten gemessen:

Molekulargewicht: 2.100 D

Aldehydgehalt: 0,55 µmol/mg.

3.2

Nach Inkubation einer Hämoglobinlösung mit dem Heparinfragment 3.1 (Molverhältnis Hb/Heparin = 1/5) unter den in Beispiel 2 beschriebenen Bedingungen wurde das Heparinfragment-Hämoglobin-Addukt isoliert. Es wurde ein

P 50-Wert von 8,14 kPa (61,1 mmHg) gemessen.

Mono S: Peakgruppenverhältnis (vgl. Abb. 5) 68, 2/31,8 (Umsatzrate: 63,8 %).

Beispiel 4

Herstellung eines Heparinfragmentes aus "High Affinity"-Heparin

4.1

Handelsübliches Heparin kann durch Affinitätschromatographie über an Sepharose gekoppeltes Antithrombin III (AT III), einem im menschlichen Plasma vorkommenden Glykoprotein, in drei unterschiedliche Fraktionen aufgetrennt werden, die sich in der Stärke ihrer Bindung zu AT III (no affinity, low affinity und high affinity-heparin) unterscheiden (J. Hopwood, M. Höök, A. Linker, U. Lindahl, FEBS-Letters 69, 51-54 (1976).

Die analytische oder präparative Auftrennung von Heparin in die genannten Fraktionen ist dem Fachmann geläufig.

Es zeigte sich, daß sich insbesondere die an AT III mit hoher Affinität bindende Fraktion (high affinity-heparin) bzw. dessen Spaltprodukte (Verfahren gem. Beispiel 1) mit Hämoglobin in gleicher Weise umsetzen wie dies mit handelsüblichem Heparin beobachtet wird.

Es wurden für das betreffende Heparinfragment folgende Daten gemessen:

Molekulargewicht: 7.000 D

Aldehydgehalt: 0,45 µmol/mg

4.2

Nach Inkubation einer Hämoglobinlösung mit dem Heparinfragment 4.1 (Molverhältnis Hb/Heparin = 1/5) unter den in Beispiel 2 beschriebenen Bedingungen wurde das Hämoglobin-Heparinfragment-Addukt isoliert. Es wurde ein P 50-Wert von 5,98 kPa (44,9 mmHg) gemessen.

Chromatographie über Mono S:Peakgruppenverhältnis (vgl. Abb. 5) 68,2/31,8 (Umsatzrate: 57,7 %).

Beispiel 5

Herstellung eines Heparingfragments aus Rinderlungenheparin ("Typ B"-Heparin)

5.1
Rinderlungenheparin (Hepar Industries, Inc., Ohio, USA) wurde gemäss Beispiel 1 einem Abbau mit $HNO_2$ unterworfen.
Mit dem entstandenen Produkt wurden folgende analytische Daten erhalten:
Molekulargewicht: 2.600 D
Aldehydgehalt: 0,44 µmol/mg.
5.2
Nach Inkubation einer Hämoglobinlösung mit dem Heparinfragment 5.1 (Molverhältnis Hb/Heparin = 1/1) unter den in Beispiel 2 beschriebenen Bedingungen wurde das Heparinfragment-Hämoglobin-Addukt isoliert. Es wurde ein P 50-Wert von 3,26 kPa (24,45 mmHg) gemessen.
Chromatographie über S: Peakgruppenverhältnis 62/38 (Umsatzrate: 56,8 %).

Beispiel 6

Herstellung eines Heparinfragmentes aus Heparin der Salbenqualitätsstufe

6.1
Heparin von Salbenqualität wurde gemäss Beispiel 1 abgebaut.
Folgende Produktdaten wurden erhalten:
Molekulargewicht: 2.600 D
Aldehydgehalt: 0,44 µmol/mg
6.2
Nach Inkubation einer Hämoglobinlösung mit dem Heparinfragment 6.1 (Molverhältnis Hb/Heparin = 1/5) unter den in Beispiel 2 beschriebenen Bedingungen wurde das Heparinfragment-Hämoglobin-Addukt isoliert. Es wurde ein P 50-Wert von 5,32 kPa (40 mmHg) gemessen.
Chromatographie über Mono S: Peakgruppenverhältnis 60/40 (Umsatzrate: 54,5 %).

Beispiel 7

Herstellung von Monosaccharidfragmenten aus Heparanbzw. Dermatansulfat

7.1
Auch enders sulfatierte Glykosaminoglykane sind mit den in den Beispielen 1 - 3 beschriebenen Verfahren abbaubar und als Effektoren für die Umsetzung mit Hämoglobin zu verwenden.
Gemäss dem Beispiel 1 wurden a) Heparansulfat bzw. b) Dermatansulfat (Sigma Chemie GmbH., St. Louis, USA/Calbiochem) fragmentiert.
Folgende Produktdaten wurden erhalten:

| | |
|---|---|
| Molekulargewicht: | a) 400 - 650 D |
| | b) 500 - 700 D |
| Aldehydgehalt: | a) 2,2 µmol/mg |
| | b) 1,8 µmol/mg |

7.2
Nach Inkubation einer Hämoglobinlösung mit den Fragmenten 7.1 unter den in Beispiel 2 beschriebenen Bedingungen wurden die Hämoglobin-Fragment-Addukte isoliert. Es wurden P 50-Werte von a) 5,58 kPa (41,9 mmHg) bzw. b) 5,25 kPa (33,4 mmHg) gemessen.
Chromatographie über Mono S: Peakgruppenverhältnis 65/35 (Umsatzrate: 60,22 %).

Beispiel 8

8.1 Sulfatierung von Heparin
In handelsübliches Heparin-Natrium mit 162 IE/mg (gem. USP XX) werden zur Erhöhung des Sulfatgehaltes nach der von C.R. Ricketts gegebenen Vorschrift, vgl. Biochem. J.51, 129-133 (1952) "Dextrane sulphate - a synthetic analogue of heparin", Sulfatgruppen eingeführt, dieses "supersulfatierte" Heparin isoliert und in einem Folgeschritt gem. Beispiel 1 mit $HNO_2$ umgesetzt.

In eine Lösung von 200 ml über Molekularsieb getrocknetem Pyridin werden unter starkem Rühren 44 ml Chlorsulfonsäure zugetropft. Dabei muss wegen der starken Wärmeentwicklung mit Trockeneis/Ethanol gekühlt werden. Die Temperatur wird auf 65 °C erhöht und 30 g feingepulvertes, in Vakuum bei 50 °C getrocknetes, Heparin unter starkem Rühren portionsweise zugegeben. Man hält 4 h

bei 65 bis 70 °C. Es wird auf Raumtemperatur abgekühlt und die Reaktionsmischung auf 800 ml Eis gegossen. Nun wird so lange eine 40 %ige (W/V)Natronlauge zugegeben, bis die Mischung eine dunkelrote Farbe annimmt. Es bilden sich zwei Phasen aus. Die obere Phase (Pyridin) wird im Scheidetrichter abgetrennt. Die untere Phase wird bei 4 °C mit 2 Liter gekühltem Ethanol versetzt. Dabei fällt das sulfatierte Heparin aus. Nach 12stündigem Stehenlassen bei 4 °C wird dekantiert und der Rückstand in 200 ml Wasser aufgenommen und bei 4 °C 48 h lang im Cuprophanschlauch gegen Wasser dialysiert. Die dialysierte Lösung wird zur Gewinnung des Heparinderivates lyophilisiert.

Ausbeute, bezogen auf Heparin: 22 g (73 %)

Analytik: freies Sulfat nicht nachweisbar

Antikoagulatorische Aktivität: 195 E/mg gem. USP XX, Schwefelgehalt: 13,1 %

Der Schwefelgehalt des Heparins, das zur Sulfatierung eingesetzt wurde, liegt bei 10,2 %.

8.2 Abbau des sulfatierten Heparins gem. 8.1.

10 g des gem. 8.1 hergestellten Heparinderivats werden nach der in Beispiel 1 gegebenen Vorschrift mit $HNO_2$ bei pH 4,0 umgesetzt, im Cuprophan-Dialyseschlauch bis zur Nitritfreiheit dialysiert und zur Gewinnung der Festsubstanz lyophilisiert. Es wurden 6,8 g des $HNO_2$-Spaltproduktes isoliert.

Die antikoagulatorische Aktivität gem. USP XX liegt bei 1,3 IE/mg.

Aldehydgehalt: 0,57 µmol/mg

Molmassenverteilung: 1.500 - 2.500 D

Mittleres Molekulargewicht: 1.700 D.

8.3

Gemäss der in Beispiel 1.3 gegebenen Vorschrift wird das gem. 8.2 erhaltene Heparinspaltprodukt mit Hämoglobinlösung umgesetzt.

250 ml deoxygeniertes Hämoglobinlysat mit einem Hämoglobingehalt von 5,3 % werden mit 0,88 g Haparinfragment 8.2 unter den in Beispiel 1 gegebenen Bedingungen 5 h bei Raumtemperatur umgesetzt. Das Reaktionsgemisch wird mit 2 Litern $N_2$-begastem 225 mM BISTRIS-Puffer, pH 7,4 (37 °C) in einer Amicon-Rührzelle diafiltriert. Die Auswertung der Sauerstoffbindungskurve ergab einen p 50-Wert von 8,91 kPa (66,8 mmHg).

## Patentansprüche

## Patentansprüche für folgende Vertragsstaaten : AT, BE, CH, DE, FR, GB, IT, LI, LU, NL, SE

1. Stromafreie Blutersatzmittel, dadurch gekennzeichnet, daß man in an sich bekannter Weise hergestellte Fragmente von sulfatierten Glykosaminoglykanen, ausgewählt aus Heparin, Heparinoiden, Dermatansulfat, Heparansulfat, sulfatiertem Chitin und/oder Chitosan, wobei die Heparinfragmente Molekulargewichte von 1000 bis 10.000 aufweisen, mit Hämoglobin, welches aus verschiedenen Spezies, wie Mensch, Schwein, Rind, gewonnen werden kann, zu stabilen, kovalent miteinander verknüpften Addukten derart umsetzt, daß deren Sauerstoff-Bindungsverhalten durch die Einstellung des Verhältnisses von Hämoglobin zu Glycosaminoglykanfragment im Bereich von 2,66 kPa (20 mmHg) bis 10,66 kPa (80 mmHg) eingestellt wird, wobei das molare Verhältnis von Hämoglobin zu Heparinfragmenten im Bereich von 1 : 0,4 bis 1 : 5 eingestellt wird.

2. Stromafreie Blutersatzmittel nach Anspruch 1, dadurch gekennzeichnet, daß das Sauerstoff-Bindungsverhalten im Bereich von 4,79 kPa (36 mmHg) bis 5,33 kPa (40 mmHg) eingestellt wird.

3. Stromafreie Blutersatzmittel nach Ansprüchen 1 oder 2, dadurch gekennzeichnet, daß die Heparinfragmente Molekulargewichte von 1000 bis 3000 aufweisen und gleichzeitig reaktive Gruppen, wie Aldehydfunktionen, tragen.

4. Stromafreie Blutersatzmittel nach Anspruch 1 - 3, dadurch gekennzeichnet, daß das molare Verhältnis von Hämoglobin zu Heparinfragmenten im Bereich von 1 : 3 bis 1 : 5 eingestellt wird, wobei als Bezugsbasis für Hämoglobin das Hämoglobintetramer und für das Heparinfragment ein mittleres Molekulargewicht von 2000 zugrunde gelegt wird.

5. Stromafreie Blutersatzmittel nach Anspruch 4, dadurch gekennzeichnet, daß der Gehalt an Heparinfragmenten zu Hämoglobin im Massenverhältnis von 1 : 7 bis 1 : 84 eingestellt wird.

6. Stromafreie Blutersatzmittel nach Anspruch 1 - 5, dadurch gekennzeichnet, daß im Falle eines durch $HNO_2$ gespaltenen Heparinfragmentes das Molverhältnis Hämoglobin/Aldehydgehalt des Fragmentes

im Verhältnis von 1 : 0,4 bis 1 : 5 bei einem mittleren Aldehydgehalt von 0,5 µmol/mg Heparinfragment eingestellt wird.

7. Verfahren zur Herstellung von stromafreien Blutersatzmitteln nach Ansprüchen 1 - 6, dadurch gekennzeichnet, daß man die sulfatierten Glucosaminoglykanfragmente in wässriger gepufferter Lösung im pH-Wert-Bereich von 5,5 bis 8,5 und vorzugsweise 6,1 bis 7,8 mit in an sich bekannter Weise hergestellten oxygenierten und vorzugsweise deoxygenierten Hämoglobinlösungen, enthaltend eine Hämoglobinkonzentration, bezogen auf das Hämoglobintetramere zwischen 0,5 und 40 Gew.-% und vorzugsweise 5 bis 9 Gew.-% bei einer Temperatur im Bereich von 2 bis 60 ° C und vorzugsweise 10 bis 30 ° C umsetzt, anschließend zur Aufarbeitung des Reaktionsgemisches die Hämoglobinkonzentration auf 5 bis 7 Gew.-% einstellt und gegen einen Puffer wie Tyrode-Puffer diafiltriert, und die modifizierte Hämoglobinlösung zur Sterilfiltration und Abtrennung möglicherweise enthaltener Pyrogene über eine 0,2 um-Filtrationskerze mit positivem Zetapotential filtriert.

8. Verfahren zur Herstellung von stromafreien Blutersatzmitteln nach Anspruch 7, dadurch gekennzeichnet, daß die Umsetzung bei Raumtemperatur durchgeführt wird.

9. Verfahren zur Herstellung von stromafreien Blutersatzmitteln nach Anspruch 7 und 8, dadurch gekennzeichnet, daß die Umsetzung unter Sauerstoffausschluss in einem Laborfermenter durchgeführt wird.

10. Verfahren zur Herstellung von stromafreien Blutersatzmitteln nach Anspruch 7, dadurch gekennzeichnet, daß der Puffer für die Umsetzung des Heparinfragmentes mit Hämoglobin ausgewählt ist aus Phosphat, Hydrogencarbonat oder BISTRIS-Puffer dessen Konzentrat an puffernden Ionen 0,05 bis 1 M beträgt.

11. Verfahren zur Herstellung von stromafreien Blutersatzmitteln nach Ansprüchen 1 - 6, dadurch gekennzeichnet, daß man reaktive Heparinfragmente in isotonischem BISTRIS-Puffer bei pH 7,4 und Raumtemperatur mit intakten Erythrozyten inkubiert, wobei das molare Verhältnis von Hämoglobintetramer zu Heparinfragment im Verhältnis von 1 : 0,5 bis 1 : 20 eingestellt wird.

12. Verfahren zur Herstellung von stromafreien Blutersatzmitteln nach Anspruch 11, dadurch gekennzeichnet, daß man die Heparinfragmente in an sich bekannter Weise in mit DMSO permeabilisierte Erythrozyten einschließt oder in an sich bekannter Weise in Liposomen inkorporiert und anschließend mit Erythrozyten inkubiert.

13. Verfahren zur Herstellung von stromafreien Blutersatzmitteln nach Ansprüchen 1 - 12, dadurch gekennzeichnet, daß man Heparin, Dermatansulfat, Heparansulfat, Chitin oder Chitosan vor der Spaltung zu den entsprechenden Glykosaminoglykanfragmenten in an sich bekannter Weise zu N- und O-Sulfatestern umsetzt.

14. Verfahren zur Herstellung von stromafreien Blutersatzmitteln nach Anspruch 7, dadurch gekennzeichnet, daß man Hämoglobin-Heparinfragment-Addukte mit Natriumborhydrid in an sich bekannter Weise reduziert.

15. Verfahren zur Herstellung von stromafreien Blutersatzmitteln nach Anspruch 7, dadurch gekennzeichnet, daß man Hämoglobin-Heparinfragment-Addukte mit bi- und/oder polyfunktionellen Vernetzungsmitteln und vorzugsweise in an sich bekannter Weise mit Glutardialdehyd vernetzt.

16. Verfahren zur Herstellung von stromafreien Blutersatzmitteln nach Anspruch 7, dadurch gekennzeichnet, daß man Hämoglobin-Heparinfragment-Addukte in Hämosomen in an sich bekannter Weise einschließt.

17. Pharmazeutische Zubereitung, enthaltend stromafreie Bluterstazmittel gemäß Ansprüchen 1 bis 6 zusammen mit üblichen pharmazeutischen Trägern und Hilfsstoffen.

**Patenansprüche für folgende Vertragsstaaten : ES, GR**

1. Verfahren zur Herstellung von stromafreien Blutersatzmitteln, dadurch gekennzeichnet, daß man in an

sich bekannter Weise hergestellte Fragmente von sulfatierten Glykosaminoglykanen, ausgewählt aus Heparin, Heparinoiden, Dermatansulfat, Heparansulfat, sulfatiertem Chitin und/oder Chitosan, wobei die Heparinfragmente Molekulargewichte von 1000 bis 10.000 aufweisen, mit Hämoglobin, welches aus verschiedenen Spezies, wie Mensch, Schwein, Rind, gewonnen werden kann, zu stabilen, kovalent miteinander verknüpften Addukten derart umsetzt, daß deren Sauerstoff-Bindungsverhalten durch die Einstellung des Verhältnisses von Hämoglobin zu Glycosaminoglykanfragment im Bereich von 2,66 kPa (20 mmHg) bis 10,66 kPa (80 mmHg) eingestellt wird, wobei das molare Verhältnis von Hämoglobin zu Heparinfragmenten im Bereich von 1 : 0,4 bis 1 : 5 eingestellt wird.

**2.** Verfahren nach Anspruch 1, dadurch gekennzeichnet, daß das Sauerstoff-Bindungsverhalten im Bereich von 4,79 kPa (36 mmHg) bis 5,33 kPa (40 mmHg) eingestellt wird.

**3.** Verfahren nach Ansprüchen 1 oder 2, dadurch gekennzeichnet, daß die Heparinfragmente Molekulargewichte von 1000 bis 3000 aufweisen und gleichzeitig reaktive Gruppen, wie Aldehydfunktionen, tragen.

**4.** Verfahren nach Anspruch 1 - 3, dadurch gekennzeichnet, daß das molare Verhältnis von Hämoglobin zu Heparinfragmenten im Bereich von 1 : 3 bis 1 : 5 eingestellt wird, wobei als Bezugsbasis für Hämoglobin das Hämoglobintetramer und für die Heparinfragmente ein mittleres Molekulargewicht von 2000 zugrunde gelegt wird.

**5.** Verfahren nach Anspruch 4, dadurch gekennzeichnet, daß der Gehalt an Heparinfragmenten zu Hämoglobin im Massenverhältnis von 1 : 7 bis 1 : 84 eingestellt wird.

**6.** Verfahren nach Anspruch 1 - 5, dadurch gekennzeichnet, daß im Falle eines durch $HNO_2$ gespaltenen Heparinfragmentes das Molverhältnis Hämoglobin/Aldehydgehalt des Fragmentes im Verhältnis von 1 : 0,4 bis 1 : 5 bei einem mittleren Aldehydgehalt von 0,5 $\mu$mol/mg Heparinfragment eingestellt wird.

**7.** Verfahren zur Herstellung von stromafreien Blutersatzmitteln nach Ansprüchen 1 - 6, dadurch gekennzeichnet, daß man die sulfatierten Glucosaminoglykanfragmente in wässriger gepufferter Lösung im pH-Wert-Bereich von 5,5 bis 8,5 und vorzugsweise 6,1 bis 7,8 mit in an sich bekannter Weise hergestellten oxygenierten und vorzugsweise deoxygenierten Hämoglobinlösungen, enthaltend eine Hämoglobinkonzentration, bezogen auf das Hämoglobintetramere zwischen 0,5 und 40 Gew.-% und vorzugsweise 5 bis 9 Gew.-% bei einer Temperatur im Bereich von 2 bis 60 °C und vorzugsweise 10 bis 30 °C umsetzt, anschließend zur Aufarbeitung des Reaktionsgemisches die Hämoglobinkonzentration auf 5 bis 7 Gew.-% einstellt und gegen einen Puffer, wie Tyrode-Puffer, diafiltriert, und die modifizierte Hämoglobinlösung zur Sterilfiltration und Abtrennung möglicherweise enthaltener Pyrogene über eine 0,2 um-Filtrationskerze mit positivem Zetapotential filtriert.

**8.** Verfahren zur Herstellung von stromafreien Blutersatzmitteln nach Anspruch 7, dadurch gekennzeichnet, daß die Umsetzung bei Raumtemperatur durchgeführt wird.

**9.** Verfahren zur Herstellung von stromafreien Blutersatzmitteln nach Ansprüchen 7 und 8, dadurch gekennzeichnet, daß die Umsetzung unter Sauerstoffausschluss in einem Laborfermenter durchgeführt wird.

**10.** Verfahren zur Herstellung von stromafreien Blutersatzmitteln nach Anspruch 7, dadurch gekennzeichnet, daß der Puffer für die Umsetzung des Heparinfragmentes mit Hämoglobin ausgewählt ist aus Phosphat-, Hydrogencarbonat- oder BISTRIS-Puffer dessen Konzentration an puffernden Ionen 0,05 bis 1 M beträgt.

**11.** Verfahren zur Herstellung von stromafreien Blutersatzmitteln nach Ansprüchen 1 - 6, dadurch gekennzeichnet, daß man reaktive Heparinfragmente in isotonischem BISTRIS-Puffer bei pH 7,4 und Raumtemperatur mit intakten Erythrozyten inkubiert, wobei das molare Verhältnis von Hämoglobintetramer zu Heparinfragmenten im Verhältnis von 1 : 0,5 bis 1 : 20 eingestellt wird.

**12.** Verfahren zur Herstellung von stromafreien Blutersatzmitteln nach Anspruch 11, dadurch gekennzeichnet, daß man die Heparinfragmente in an sich bekannter Weise in mit DMSO permeabilisierte Erythrozyten einschließt oder in an sich bekannter Weise in Liposomen inkorporiert und anschließend

mit Erythrozyten inkubiert.

13. Verfahren zur Herstellung von stromafreien Blutersatzmitteln nach Ansprüchen 1 - 12, dadurch gekennzeichnet, daß man Heparin, Dermatansulfat, Heparansulfat, Chitin oder Chitosan vor der Spaltung zu den entsprechenden Glykosaminoglykanfragmenten in an sich bekannter Weise zu N- und O-Sulfatestern umsetzt.

14. Verfahren zur Herstellung von stromafreien Blutersatzmitteln nach Anspruch 7, dadurch gekennzeichnet, daß man Hämoglobin-Heparinfragment-Addukte mit Natriumborhydrid in an sich bekannter Weise reduziert.

15. Verfahren zur Herstellung von stromafreien Blutersatzmitteln nach Anspruch 7, dadurch gekennzeichnet, daß man Hämoglobin-Heparinfragment-Addukte mit bi- und/oder polyfunktionellen Vernetzungsmitteln und vorzugsweise in an sich bekannter Weise mit Glutardialdehyd vernetzt.

16. Verfahren zur Herstellung von stromafreien Blutersatzmitteln nach Anspruch 7, dadurch gekennzeichnet, daß man Hämoglobin-Heparinfragment-Addukte in Hämosomen in an sich bekannter Weise einschließt.

## Claims

**Claims for the following Contracting States : AT, BE, CH, DE, FR, GB, IT, LI, LU, NL, SE**

1. A stroma-free blood substitute, characterized in that fragments, prepared in a per se known manner, of sulfated glycosaminoglycans, selected from heparin, heparinoids, dermatan sulfate, heparan sulfate, sulfated chitin and/or chitosan, wherein the heparin fragments have molecular weights of from 1,000 to 10,000, are reacted with haemoglobin which may be recovered from various species such as human, bovine, porcine, to form stable covalently linked adducts in such a manner that by pre-determining the ratio of haemoglobin to glycosaminoglycan fragment the oxygen binding property thereof is adjusted to be within the range of from 2.66 kPa (20 mmHg) to 10.66 kPa (80 mmHg), the molar ratio of haemoglobin to heparin fragments being adjusted to be within the range of from 1:0.4 to 1:5.

2. The stroma-free blood substitute according to claim 1, characterized in that the oxygen binding property thereof is adjusted to be within the range of from 4.79 kPa (36 mmHg) to 5.33 kPa (40 mmHg).

3. The stroma-free blood substitute according to claims 1 or 2, characterized in that the heparin fragments have molecular weights of from 1,000 to 3,000 and at the same time bear reactive groups such as aldehyde functions.

4. The stroma-free blood substitute according to claims 1 to 3, characterized in that the molar ratio of haemoglobin to heparin fragments is adjusted to be within the range of from 1:3 to 1:5, the reference standard for haemoglobin being the haemoglobin tetramer and the reference standard for the heparin fragment being an average molecular weight of 2,000.

5. The stroma-free blood substitute according to claim 4, characterized in that the content of heparin fragments relative to haemoglobin is adjusted to a mass ratio of from 1:7 to 1:84.

6. The stroma-free blood substitute according to claims 1 to 5, characterized in that in the case of a heparin fragment obtained by $HNO_2$ cleavage the molar ratio of haemoglobin to aldehyde content of the fragment is adjusted to be within the range of from 1:0.4 to 1:5 at an average aldehyde content of 0.5 $\mu$mol/mg of heparin fragment.

7. A process for preparing a stroma-free blood substitute according to claims 1 to 6, characterized in that the sulfated glycosaminoglycan fragments are reacted in an aqueous buffered solution in a pH range of from 5.5 to 8.5, and preferably from 6.1 to 7.8, with oxygenated, and preferably deoxygenated, haemoglobin solutions prepared in a per se known manner and containing a haemoglobin concentration, based on the haemoglobin tetramer, of between 0.5 and 40% by weight, and preferably of from 5 to 9% by weight, at a temperature of from 2 °C to 60 °C, and preferably from 10 °C to 30 °C, then

for the work-up of the reaction mixture the haemoglobin concentration is adjusted to from 5 to 7% by weight and the mixture is diafiltrated against a buffer such as Tyrode's buffer, and the modified haemoglobin solution is filtered through a 0.2 µm filter candle having a positive zeta potential for sterile filtration and removal of possibly contained pyrogens.

**8.** The process for preparing a stroma-free blood substitute according to claim 7, characterized in that the reaction is carried out at room temperature.

**9.** The process for preparing a stroma-free blood substitute according to claims 7 and 8, characterized in that the reaction is carried out in the absence of oxygen in a laboratory fermenter.

**10.** The process for preparing a stroma-free blood substitute according to claim 7, characterized in that the buffer for the reaction of the heparin fragment with haemoglobin is selected from the group of phosphate, hydrogen carbonate and BISTRIS buffers, the concentration of buffering ions of which is from 0.05 to 1M.

**11.** The process for preparing a stroma-free blood substitute according to claims 1 to 6, characterized in that reactive heparin fragments are incubated in isotonic BISTRIS buffer at pH 7.4 and room temperature with intact erythrocytes, the molar ratio of haemoglobin tetramer to heparin fragment being adjusted to be within the range of from 1:0.5 to 1:20.

**12.** The process for preparing a stroma-free blood substitute according to claim 11, characterized in that the heparin fragments are included in a per se known manner in DMSO-permeabilized erythrocytes or incorporated in a per se known manner in liposomes and then incubated with erythrocytes.

**13.** The process for preparing a stroma-free blood substitute according to claims 1 to 12, characterized in that, prior to the degradation to the respective glycosaminoglycan fragments, heparin, dermatan sulfate, heparan sulfate, chitin or chitosan are reacted in a per se known manner to form N- and O-sulfate esters.

**14.** The process for preparing a stroma-free blood substitute according to claim 7, characterized in that haemoglobin-heparin fragment adducts are reduced with sodium borohydride in a per se known manner.

**15.** The process for preparing a stroma-free blood substitute according to claim 7, characterized in that haemoglobin-heparin fragment adducts are cross-linked with bi- and/or polyfunctional cross-linking agents, and preferably in a per se known manner with glutardialdehyde.

**16.** The process for preparing a stroma-free blood substitute according to claim 7, characterized in that haemoglobin-heparin fragment adducts are included in haemosomes in a per se known manner.

**17.** A pharmaceutical composition, containing stroma-free blood substitutes according to claims 1 to 6 together with conventional pharmaceutical carriers and excipients.

**Claims for the following Contracting States : ES, GR**

**1.** A process for preparing a stroma-free blood substitute, characterized in that fragments, prepared in a per se known manner, of sulfated glycosaminoglycans, selected from heparin, heparinoids, dermatan sulfate, heparan sulfate, sulfated chitin and/or chitosan, wherein the heparin fragments have molecular weights of from 1,000 to 10,000, are reacted with haemoglobin which may be recovered from various species such as human, bovine, porcine, to form stable covalently linked adducts in such a manner that by pre-determining the ratio of haemoglobin to glycosaminoglycan fragment the oxygen binding property thereof is adjusted to be within the range of from 2.66 kPa (20 mmHg) to 10.66 kPa (80 mmHg), the molar ratio of haemoglobin to heparin fragments being adjusted to be within the range of from 1:0.4 to 1:5.

**2.** The process according to claim 1, characterized in that the oxygen binding property thereof is adjusted to be within the range of from 4.79 kPa (36 mmHg) to 5.33 kPa (40 mmHg).

3. The process according to claims 1 or 2, characterized in that the heparin fragments have molecular weights of from 1,000 to 3,000 and at the same time bear reactive groups such as aldehyde functions.

4. The process according to claims 1 to 3, characterized in that the molar ratio of haemoglobin to heparin fragments is adjusted to be within the range of from 1:3 to 1:5, the reference standard for haemoglobin being the haemoglobin tetramer and the reference standard for the heparin fragment being an average molecular weight of 2,000.

5. The process according to claim 4, characterized in that the content of heparin fragments relative to haemoglobin is adjusted to a mass ratio of from 1:7 to 1:84.

6. The process according to claims 1 to 5, characterized in that in the case of a heparin fragment obtained by $HNO_2$ cleavage the molar ratio of haemoglobin to aldehyde content of the fragment is adjusted to be within the range of from 1:0.4 to 1:5 at an average aldehyde content of 0.5 $\mu$mol/mg of heparin fragment.

7. A process for preparing a stroma-free blood substitute according to claims 1 to 6, characterized in that the sulfated glycosaminoglycan fragments are reacted in an aqueous buffered solution in a pH range of from 5.5 to 8.5, and preferably from 6.1 to 7.8, with oxygenated, and preferably deoxygenated, haemoglobin solutions prepared in a per se known manner and containing a haemoglobin concentration, based on the haemoglobin tetramer, of between 0.5 and 40% by weight, and preferably of from 5 to 9% by weight, at a temperature of from 2 °C to 60 °C, and preferably from 10 °C to 30 °C, then for the work-up of the reaction mixture the haemoglobin concentration is adjusted to from 5 to 7% by weight and the mixture is diafiltrated against a buffer such as Tyrode's buffer, and the modified haemoglobin solution is filtered through a 0.2 $\mu$m filter candle having a positive zeta potential for sterile filtration and removal of possibly contained pyrogens.

8. The process for preparing a stroma-free blood substitute according to claim 7, characterized in that the reaction is carried out at room temperature.

9. The process for preparing a stroma-free blood substitute according to claims 7 and 8, characterized in that the reaction is carried out in the absence of oxygen in a laboratory fermenter.

10. The process for preparing a stroma-free blood substitute according to claim 7, characterized in that the buffer for the reaction of the heparin fragment with haemoglobin is selected from the group of phosphate, hydrogen carbonate and BISTRIS buffers, the concentration of buffering ions of which is from 0.05 to 1M.

11. The process for preparing a stroma-free blood substitute according to claims 1 to 6, characterized in that reactive heparin fragments are incubated in isotonic BISTRIS buffer at pH 7.4 and room temperature with intact erythrocytes, the molar ratio of haemoglobin tetramer to heparin fragment being adjusted to be within the range of from 1:0.5 to 1:20.

12. The process for preparing a stroma-free blood substitute according to claim 11, characterized in that the heparin fragments are included in a per se known manner in DMSO-permeabilized erythrocytes or incorporated in a per se known manner in liposomes and then incubated with erythrocytes.

13. The process for preparing a stroma-free blood substitute according to claims 1 to 12, characterized in that, prior to the degradation to the respective glycosaminoglycan fragments, heparin, dermatan sulfate, heparan sulfate, chitin or chitosan are reacted in a per se known manner to form N- and O-sulfate esters.

14. The process for preparing a stroma-free blood substitute according to claim 7, characterized in that haemoglobin-heparin fragment adducts are reduced with sodium borohydride in a per se known manner.

15. The process for preparing a stroma-free blood substitute according to claim 7, characterized in that haemoglobin-heparin fragment adducts are cross-linked with bi- and/or polyfunctional cross-linking

agents, and preferably in a *per se* known manner with glutardialdehyde.

16. The process for preparing a stroma-free blood substitute according to claim 7, characterized in that haemoglobin-heparin fragment adducts are included in haemosomes in a *per se* known manner.

**Revendications**

**Revendications pour les Etats contractants suivants : AT, BE, CH, DE, FR, GB, IT, LI, LU, NL, SE**

1. Substituts du sang exempts de stroma, caractérisés en ce que l'on fait réagir des fragments de glycosaminoglycannes sulfatés préparés de façon connue, choisis parmi l'héparine, les héparinoïdes, le dermatane sulfate, l'héparane sulfate, la chitine et/ou le chitosane sulfatés, les fragments d'héparine présentant des poids moléculaires de 1000 à 10 000, avec de l'hémoglobine pouvant être obtenue à partir de diverses espèces, telles que l'homme, le porc, un bovin, en formant des produits d'addition stables liés de façon covalente , de telle manière que leur affinité pour l'oxygène s'établisse dans le domaine de 2,66 kPa (20 mm de Hg) à 10,66 kPa (80 mm de Hg) par ajustement du rapport hémoglobine à fragment de glycosaminoglycanne, le rapport molaire de l'hémoglobine aux fragments d'héparine s'établissant dans la gamme de 1:0,4 à 1:5.

2. Substituts du sang exempts de stroma selon la revendication 1, caractérisés en ce que l'affinité pour l'oxygène est ajustée dans le domaine de 4,79 kPa (36 mm de Hg) à 5,33 kPa (40 mm de Hg).

3. Substituts du sang exempts de stroma selon la revendication 1 ou 2, caractérisés en ce que les fragments d'héparine présentent des poids moléculaires de 1000 à 3000 et sont en même temps porteurs de groupes réactifs, tels que des fonctions aldéhyde.

4. Substituts du sang exempts de stroma selon les revendications 1 à 3, caractérisés en ce que le rapport molaire de l'hémoglobine aux fragments d'héparine est ajusté dans le domaine de 1:3 à 1:5, l'hémoglobine tétramère étant retenue comme base de référence pour l'hémoglobine et un poids moléculaire moyen de 2000 étant retenu comme base de référence pour le fragment d'héparine.

5. Substituts du sang exempts de stroma selon la revendication 4, caractérisé en ce que la teneur en fragments d'héparine par rapport à l'hémoglobine est ajustée dans la proportion de 1:7 à 1:84.

6. Substituts du sang exempts de stroma selon les revendications 1 à 5, caractérisés en ce que, dans le cas d'un fragment d'héparine scindé par $HNO_2$, le rapport molaire hémoglobine/teneur en aldéhyde du fragment est ajusté dans un rapport de 1:04 à 1:5 pour une teneur moyenne en aldéhyde de 0,5 µmole/mg de fragment d'héparine.

7. Procédé de préparation de substituts du sang exempts de stroma selon les revendications 1 à 6, caractérisé en ce que l'on fait réagir les fragments de glycosaminoglycannes sulfatés en solution aqueuse tamponnée dans le domaine de pH de 5,5 à 8,5 et, de préférence de 6,1 à 7,8, avec des solutions d'hémoglobine oxygénées et, de préférence désoxygénées, préparées de façon connue en soi, contenant une concentration d'hémoglobine, rapportée à l'hémoglobine tétramère, entre 0,5 et 40% en poids et de préférence de 5 à 9% en poids à une température dans la plage de 2 à 60 ° C et, de préférence de 10 à 30 ° C, que pour le traitement du mélange réactionnel, on ajuste ensuite la concentration d'hémoglobine à 5 à 7% en poids et le dialyse contre un tampon, tel que le tampon à la tyrode, et que la solution d'hémoglobine modifiée est filtrée à travers une bougie filtrante de 2 µm avec un potentiel zêta positif en vue de la filtration stérile et de la séparation des substances pyrogènes éventuellement présentes.

8. Procédé de préparation de substituts du sang exempts de stroma selon la revendication 7, caractérisé en ce que la réaction est conduite à la température ambiante.

9. Procédé de préparation de substituts du sang selon les revendications 7 et 8, caractérisé en ce que la réaction est conduite à l'abri de l'oxygène dans un fermenteur de laboratoire.

10. Procédé de préparation de substituts du sang selon la revendication 7, caractérisé en ce que le tampon pour la réaction du fragment d'héparine avec l'hémoglobine est choisi parmi les tampons au phosphate,

à l'hydrogénocarbonate ou au BISTRIS dont la concentration en ions tamponnants est de 0,05 à 1 M.

11. Procédé de préparation de substituts du sang selon les revendications 1 à 6, caractérisé en ce qu'on incube des fragments d'héparine réactifs dans un tampon BIS TRIS isotonique à pH 7,4 et à la température ambiante avec des érythrocytes intacts, le rapport molaire de l'hémoglobine tétramère aux fragments d'héparine étant ajusté dans un rapport de 1:0,5 à 1:20.

12. Procédé de préparation de substituts du sang selon la revendication 11, caractérisé en ce qu'on enferme les fragments d'héparine de façon connue en soi dans des érythrocytes perméabilisés avec DMSO ou les incorpore de façon connue en soi dans des liposomes et les incube ensuite avec des érythrocytes.

13. Procédé de préparation de substituts du sang selon les revendications 1 à 12, caractérisé en ce que, préalablement à la scission en fragments de glycosaminoglycanne correspondants, on transforme l'héparine, le dermatane sulfate, l'héparane sulfate, la chitine ou le chitosane de façon connue en soi en esters O- et N-sulfuriques.

14. Procédé de préparation de substituts du sang selon la revendication 7, caractérisé en ce qu'on réduit des produits d'addition d'hémoglobine-fragment d'héparine de façon connue en soi avec du borohydrure de sodium.

15. Procédé de préparation de substituts du sang selon la revendication 7, caractérisé en ce qu'on réticule de façon connue en soi des produits d'addition d'hémoglobine-fragment d'héparine avec des agents réticulants di- et/ou polyfonctionnels et, de préférence avec du glutarodialdéhyde.

16. Procédé de préparation de substituts du sang selon la revendication 7, caractérisé en ce qu'on enferme des produits d'addition d'hémoglobine-fragment d'héparine de façon connue en soi dans des hémosomes.

17. Préparation pharmaceutique contenant des substituts du sang selon les revendications 1 à 6, ainsi que des excipients et adjuvants pharmaceutiques usuels.

**Revendications pour les Etats contractants suivants : ES, GR**

1. Procédé de préparation de substituts du sang exempts de stroma, caractérisé en ce que l'on fait réagir des fragments de glycosaminoglycannes sulfatés préparés de facon connue, choisis parmi l'héparine, les héparinoïdes, le dermatane sulfate, l'héparane sulfate, la chitine et/ou le chitosane sulfatés, les fragments d'héparine présentant des poids moléculaires de 1000 à 10.000, avec de l'hémoglobine pouvant être obtenue à partir de diverses espèces, telles que l'homme, le porc, un bovin , en formant des produits d'addition stables liés de façon covalente, de telle manière que leur affinité pour l'oxygène s'établisse dans le domaine de 2,66 kPa (20 mm de Hg) à 10,66 kPa (80 mm de Hg) par ajustement du rapport hémoglobine à fragment de glycosaminoglycanne, le rapport molaire de l'hémoglobine aux fragments d'héparine s'établissant dans la gamme de 1:0,4 à 1:5.

2. Procédé selon la revendication 1, caractérisé en ce que l'affinité pour l'oxygène est ajustée dans le domaine de 4,79 kPa (36 mm de Hg) à 5,33 kPa (40 mm de Hg).

3. Procédé selon la revendication 1 ou 2, caractérisé en ce que les fragments d'héparine présentent des poids moléculaires de 1000 à 3000 et sont en même temps porteurs de groupes réactifs, tels que des fonctions aldéhyde.

4. Procédé selon les revendications 1 à 3, caractérisé en ce que le rapport molaire de l'hémoglobine aux fragments d'héparine est ajusté dans le domaine de 1:3 à 1:5, l'hémoglobine tétramère étant retenue comme base de référence pour l'hémoglobine et un poids moléculaire moyen de 2000 étant retenu comme base de référence pour les fragments d'héparine.

5. Procédé selon la revendication 4, caractérisé en ce que la teneur en fragments d'héparine par rapport à l'hémoglobine est ajustée dans la proportion de 1:7 à 1:84.

6. Procédé selon les revendications 1 à 5, caractérise en ce que, dans le cas d'un fragment d'héparine scindé par $NHO_2$, le rapport molaire hémoglobine/teneur en aldéhyde du fragment est ajusté dans un rapport de 1:0,4 à 1:5 pour une teneur moyenne en aldéhyde de 0,5 μmole/mg de fragment d'héparine.

7. Procédé de préparation de substituts du sang exempts de stroma selon les revendications 1 à 6, caractérisé en ce que l'on fait réagir les fragments de glycosaminoglycannes sulfatés en solution aqueuse tamponnée dans le domaine de pH de 5,5 à 8,5 et, de préférence de 6,1 à 7,8, avec des solutions d'hémoglobine oxygénées et, de préférence désoxygénées, préparées de façon connue en soi, contenant une concentration d'hémoglobine, rapportée à l'hémoglobine tétramère, entre 0,5 et 40% en poids et de référence de 5 à 9% en poids à une température dans la plage de 2 à 60 ° C et, de préférence de 10 à 30 ° C, que pour le traitement du mélange réactionnel, on ajuste ensuite la concentration d'hémoglobine à 5 à 7% en poids et le dialyse contre un tampon, tel que le tampon à la tyrode,et que la solution d'hémoglobine modifiée est filtrée à travers une bougie filtrante de 0,2 μm avec un potentiel zêta positif en vue de la filtration stérile et de la séparation de substances pyrogènes éventuellement présentes.

8. Procédé de préparation de substituts du sang exempts de stroma selon la revendication 7, caractérisé en ce que la réaction est conduite à la température ambiante.

9. Procédé de préparation de substituts du sang exempts de stroma selon les revendications 7 et 8, caractérisé en ce que la réaction est conduite à l'abri de l'oxygène dans un fermenteur de laboratoire.

10. Procédé de préparation de substituts du sang exempts de stroma selon la revendication 7, caractérisé en ce que le tampon pour la réaction du fragment d'héparine avec l'hémoglobine est choisi parmi les tampons au phosphate, à l'hydrogénocarbonate ou au BISTRIS dont la concentration en ions tamponnants est de 0,05 à 1 M.

11. Procédé de préparation de substituts du sang exempts de stroma selon les revendications 1 à 6, caractérisé en ce qu'on incube des fragments d'héparine réactifs dans un tampon BIS TRIS isotonique à pH 7,4 et à la température ambiante avec des érythrocytes intacts, le rapport molaire de l'hémoglobine tétramère aux fragments d'héparine étant ajusté dans un rapport de 1:0,5 à 1:20.

12. Procédé de préparation de substituts du sang exempts de stroma selon la revendication 11, caractérisé en ce qu'on enferme les fragments d'héparine de facon connue en soi dans des érythrocytes perméabilisés avec DMSO ou les incorpore de façon connue en soi dans des liposomes et les incube ensuite avec des érythrocytes.

13. Procédé de préparation de substituts du sang exempts de stroma selon les revendications 1 à 12, caractérisé en ce que, préalablement à la scission en fragments de glycosaminoglycanne correspondants, on transforme l'héparine, le dermatane sulfate, l'héparane sulfate, la chitine ou le chitosane de façon connue en soi en esters O- et N-sulfuriques.

14. Procédé de préparation de substituts du sang exempts de stroma selon la revendication 7, caractérisé en ce qu'on réduit des produits d'addition d'hémoglobine-fragment d'héparine de facon connue en soi avec du borohydrure de sodium.

15. Procédé de préparation de substituts du sang exempts de stroma selon la revendication 7, caractérisé en ce qu'on réticule de facon connue en soi des produits d'addition d'hémoglobine-fragment d'héparine avec des agents réticulants di- et/ou polyfonctionnels et, de préférence avec du glutarodialdéhyde.

16. Procédé de préparation de substituts du sang exempts de stroma selon la revendication 7, caractérise en ce qu'on enferme des produits d'addition d'hémoglobine-fragment d'héparine de facon connue en soi dans des hémosomes.

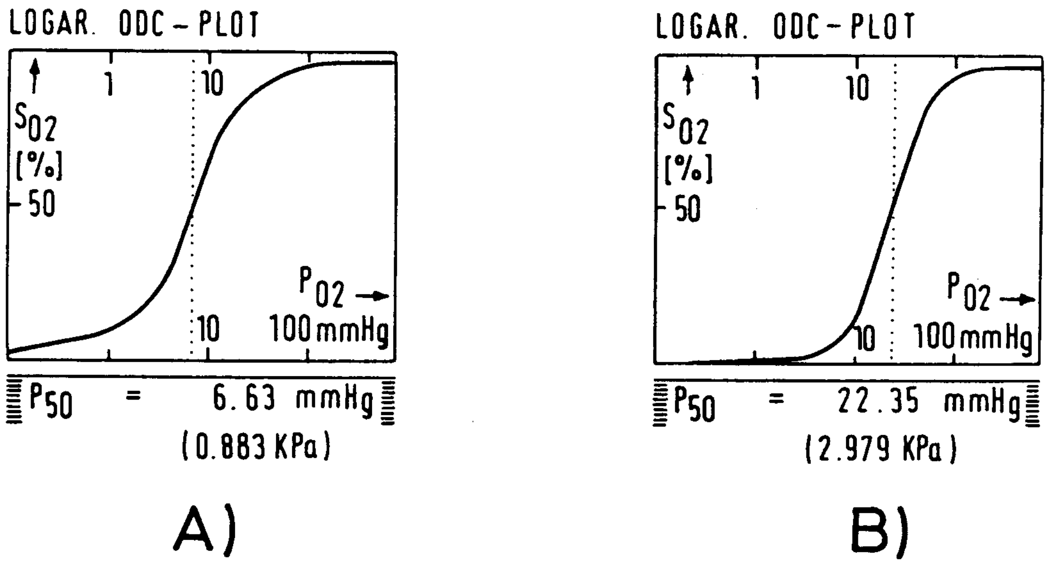


**Abb.1** Sauerstoffbindungskurven Hämoglobin-enthaltender Lösungen

A: Modifiziertes Hämoglobin

B: Nicht modifiziertes Hämoglobin

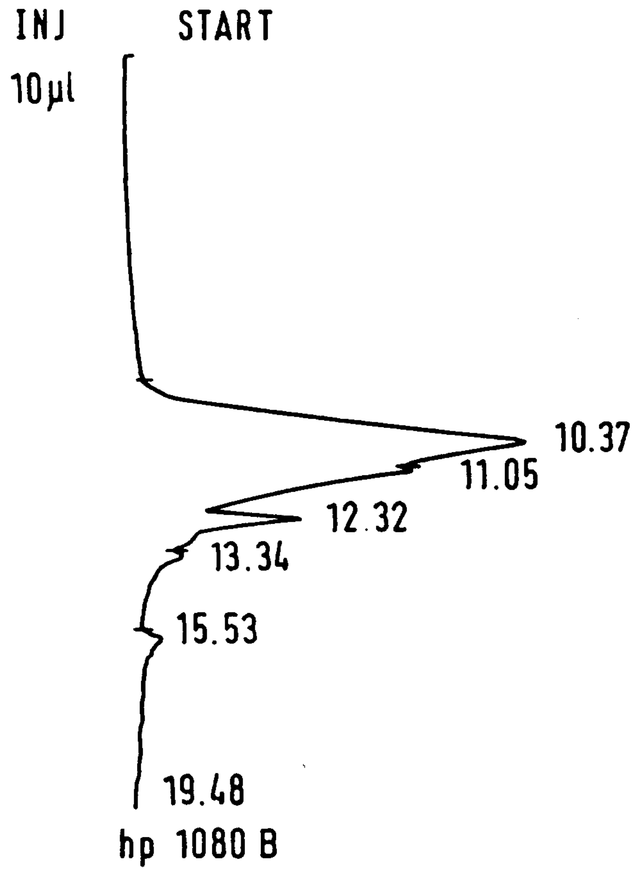


Abb.2 Elutionsprofil des gemäß 1.1 hergestellten Heparinfragmentes. Die Retentionszeit des Peakmaximums liegt bei 10,37 Minuten.

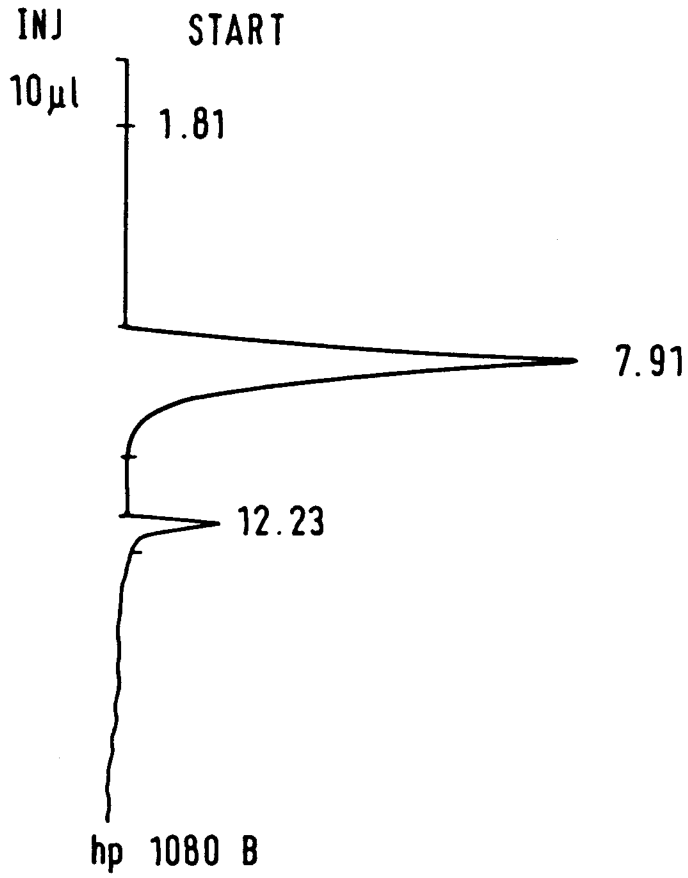


Abb.3 Elutionsprofil eines handelsüblichen Heparin-Natriumsalzes. Die Retentionszeit des Peakmaximums liegt bei 7,91 Minuten.

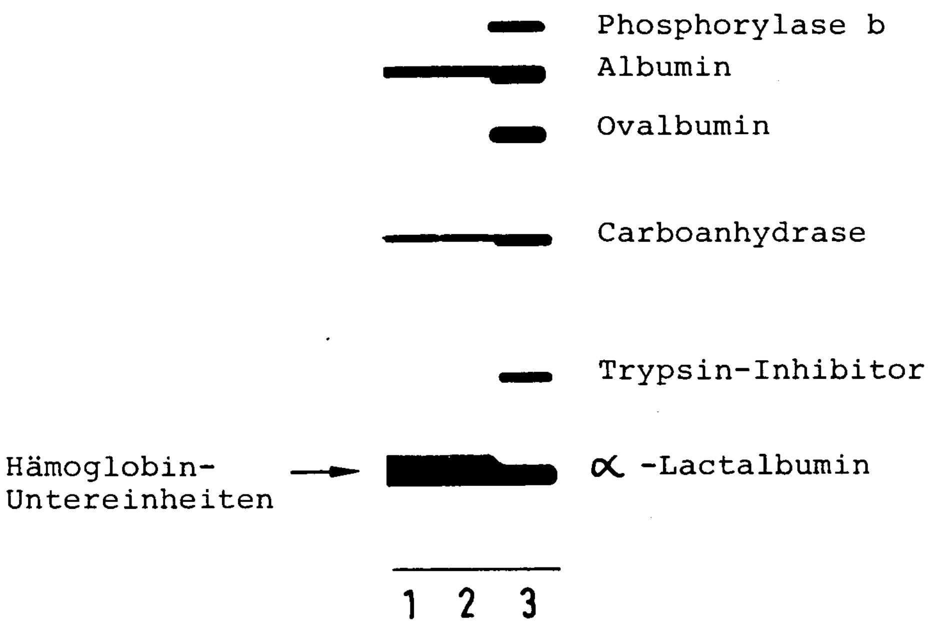


Abb.4

SDS-Polyacrylamidgelelektrophorese
Bahnbelegung: Bahn 1 Hb-Lysat, unbehandelt;
Bahn 2 Hb-Präparat, mit Heparinfragment gemäß Beispiel 1.1 umgesetzt;
Bahn 3 Molekulargewichtsmarker (Phosphorylase b - 94.000 D -, Albumin - 67.000 D -, Ovalbumin - 43.000 D -, Carboanhydrase - 30.000 D -, Trypsin-Inhibitor - 20.100 D -, α-Lactalbumin - 14.400 D -).

Abb.5

Ionenaustauschchromatographie über FPLC-Mono S

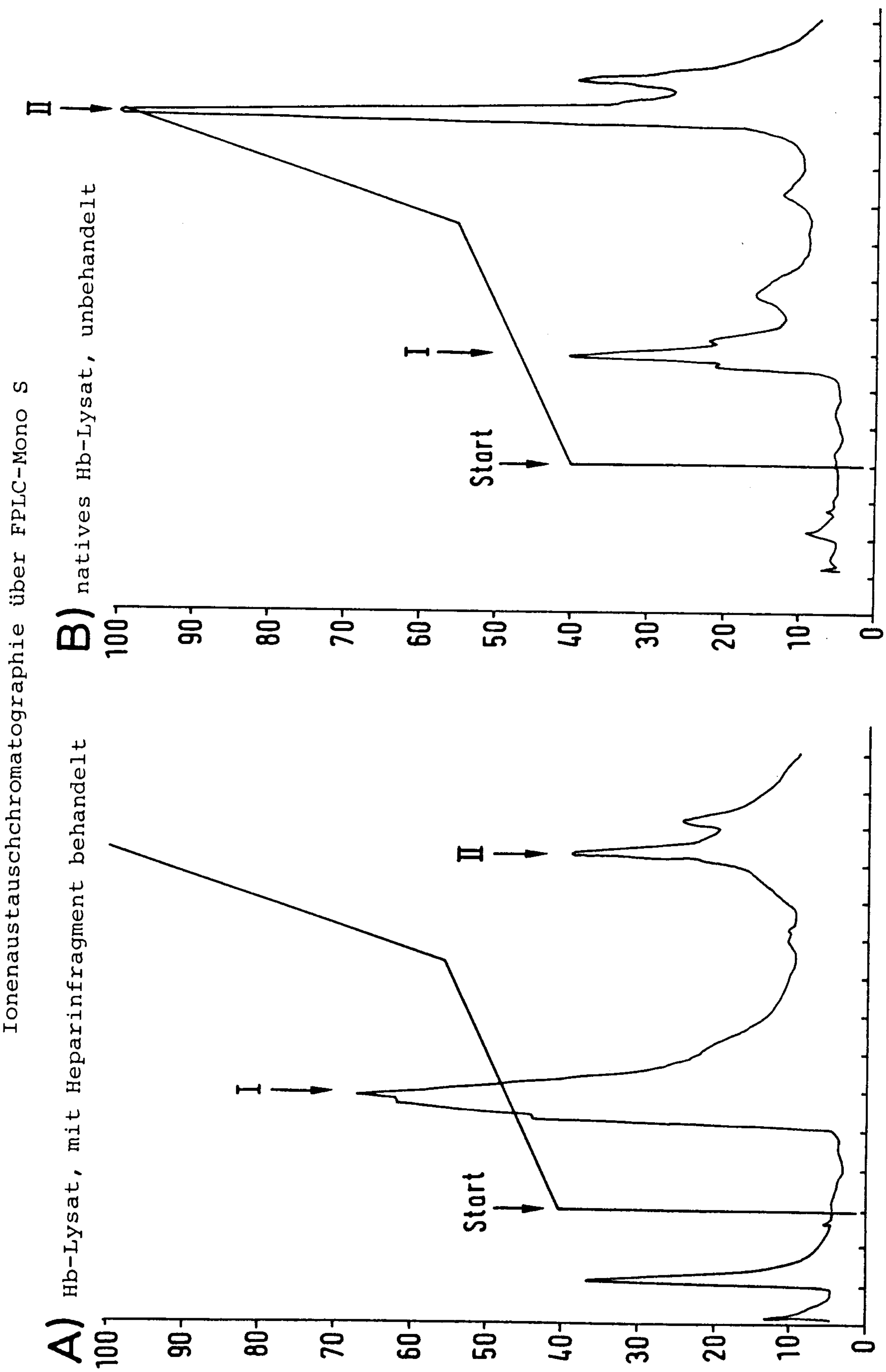

Abb.6

FPLC-Reversed-phase-Chromatographie

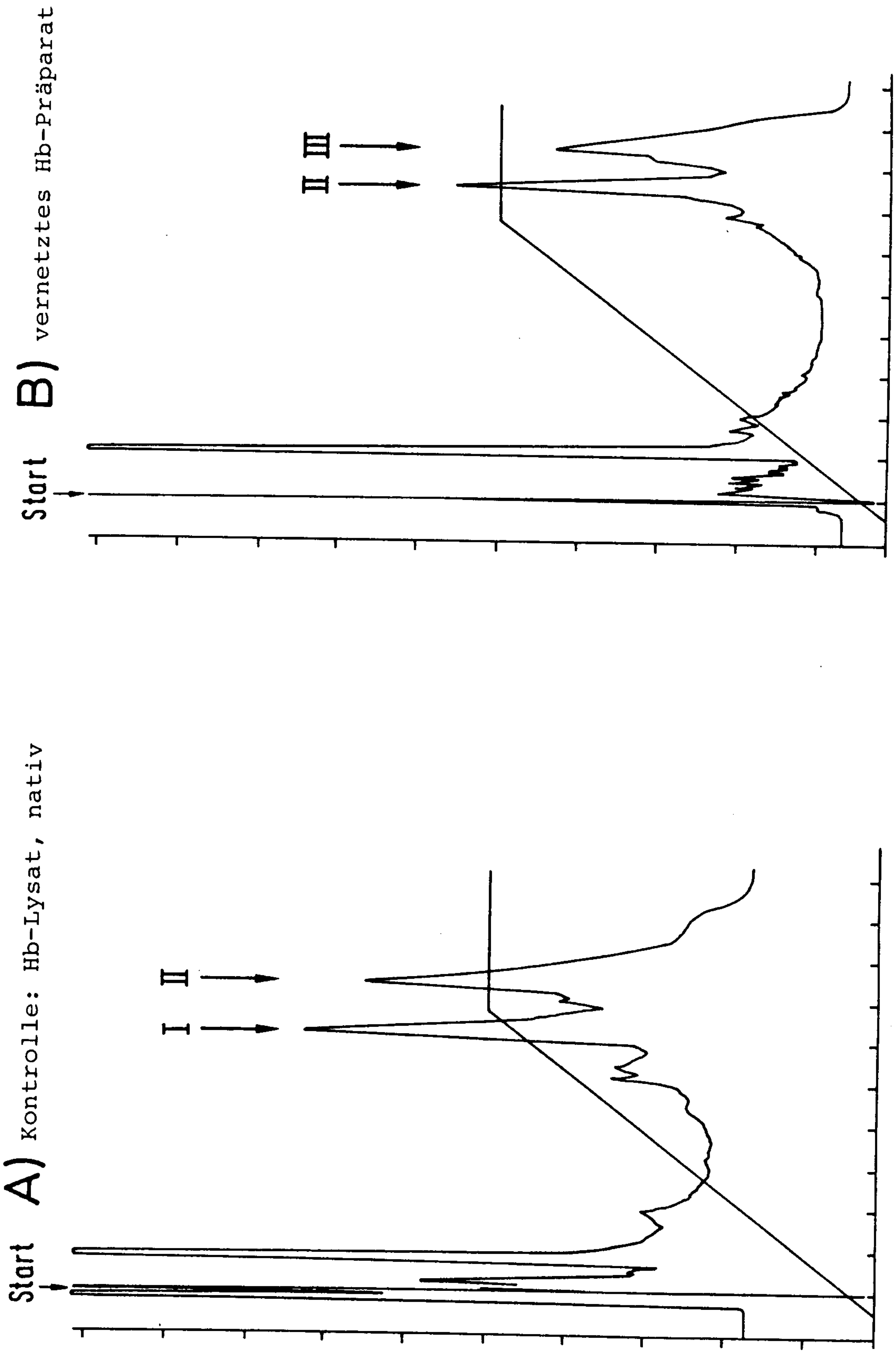

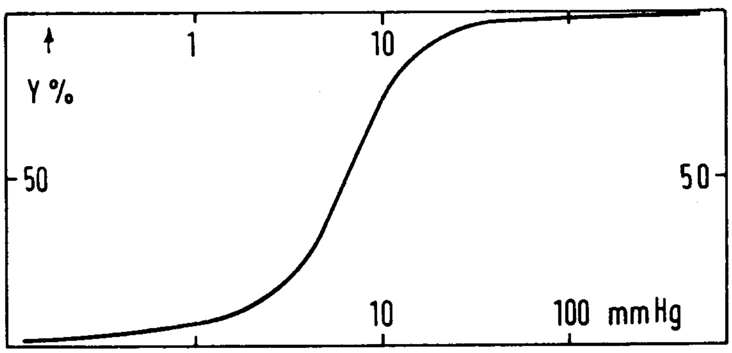


P (50) = 6,42 mmHg (0,854 KPa)
N (40-60 %) = 2,25

A)

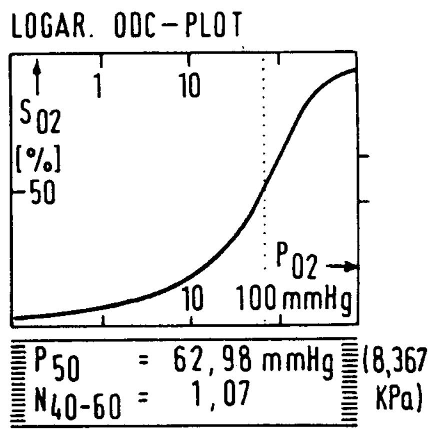


B)

Abb.7 $O_2$-Bindungskurven

A) unbehandeltes Hb-Lysat:
P (50) = 6,42 mmHg; N = 2,25

B) Hb-Präparat (Herstellung gemäß 1.3):
P (50) = 62,98 mmHg; N = 1,07

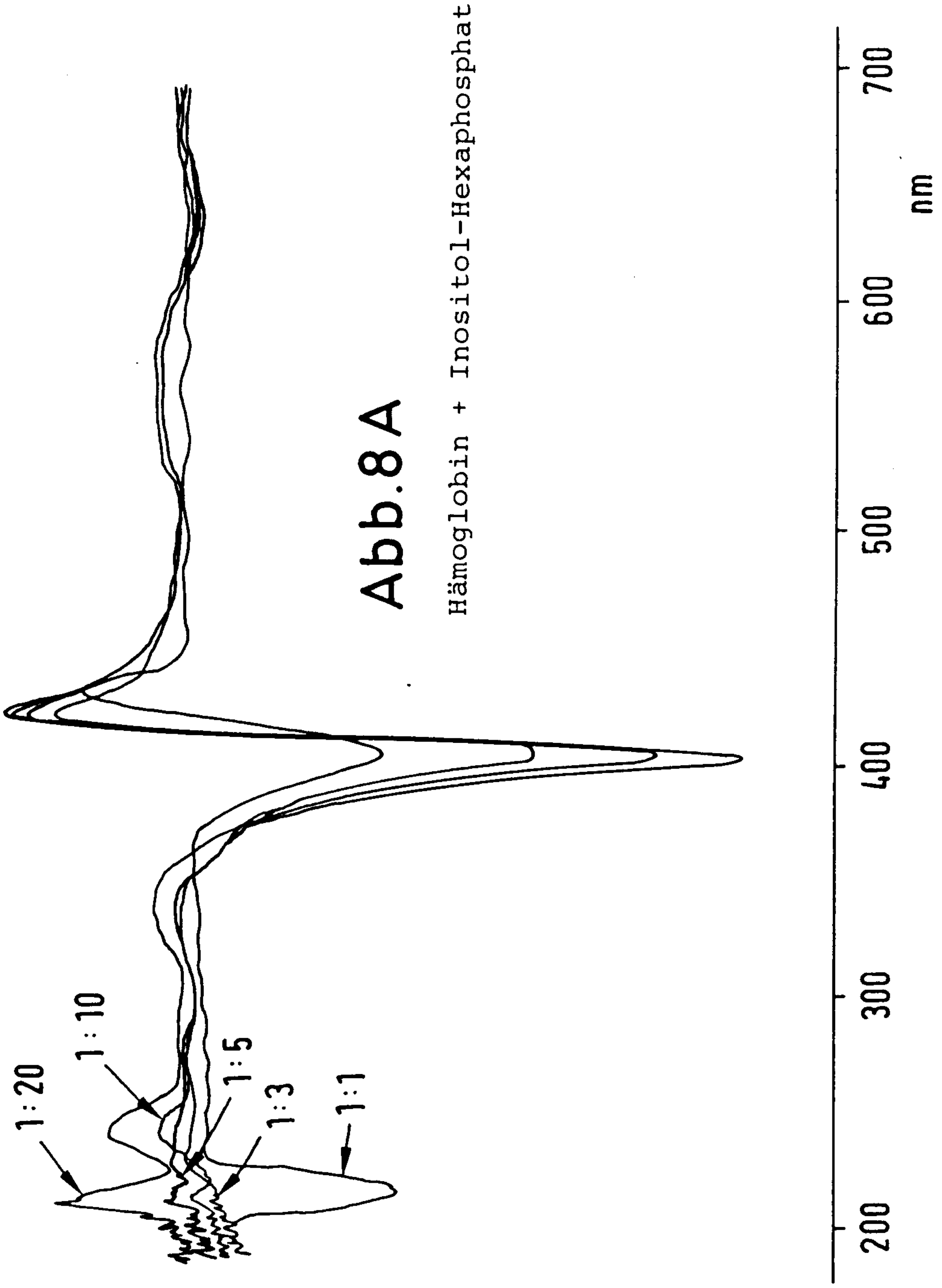


Abb.8A

Hämoglobin + Inositol-Hexaphosphat

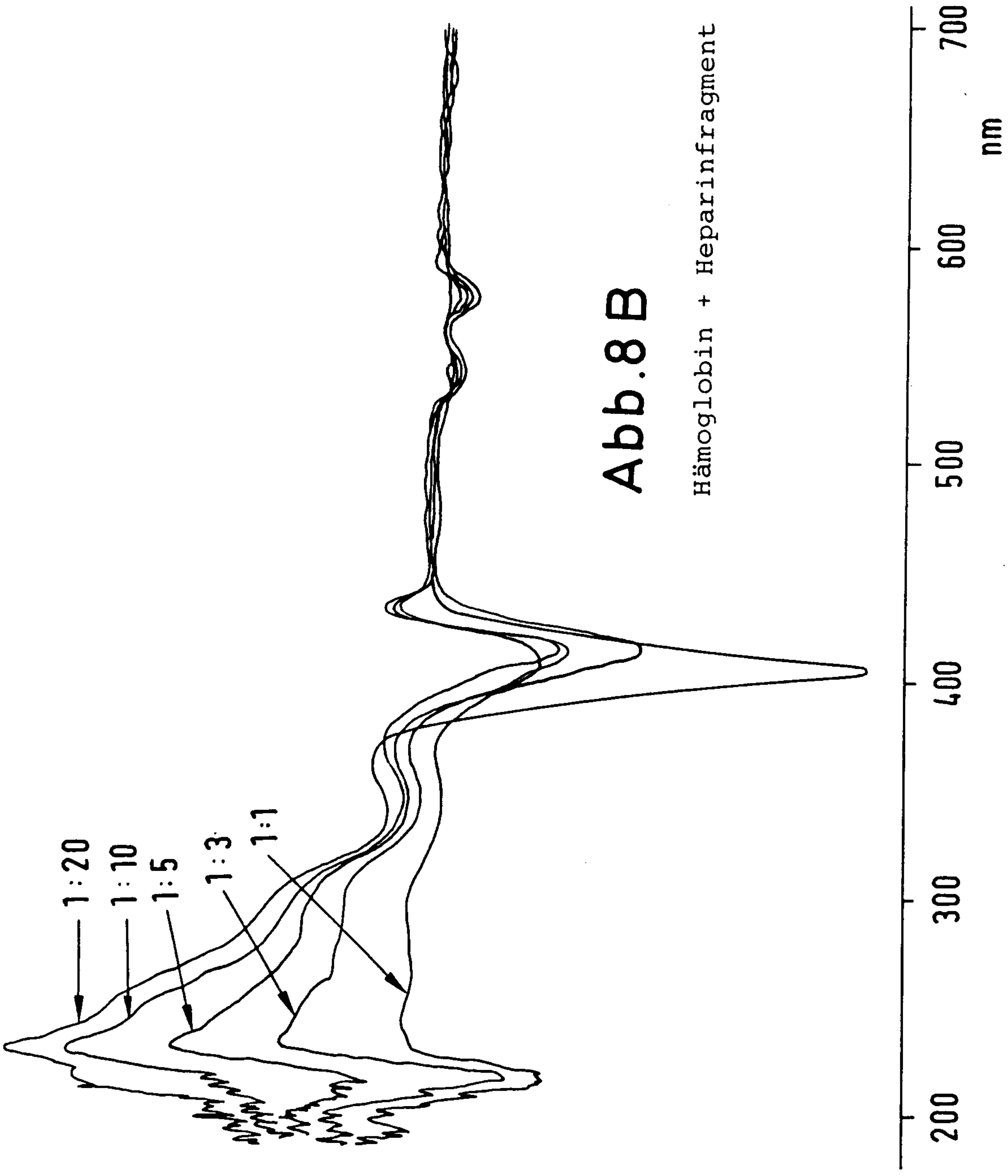


Abb. 8 B

Hämoglobin + Heparinfragment

Abb.9A

IR-Spektrum eines unfraktionierten Heparins

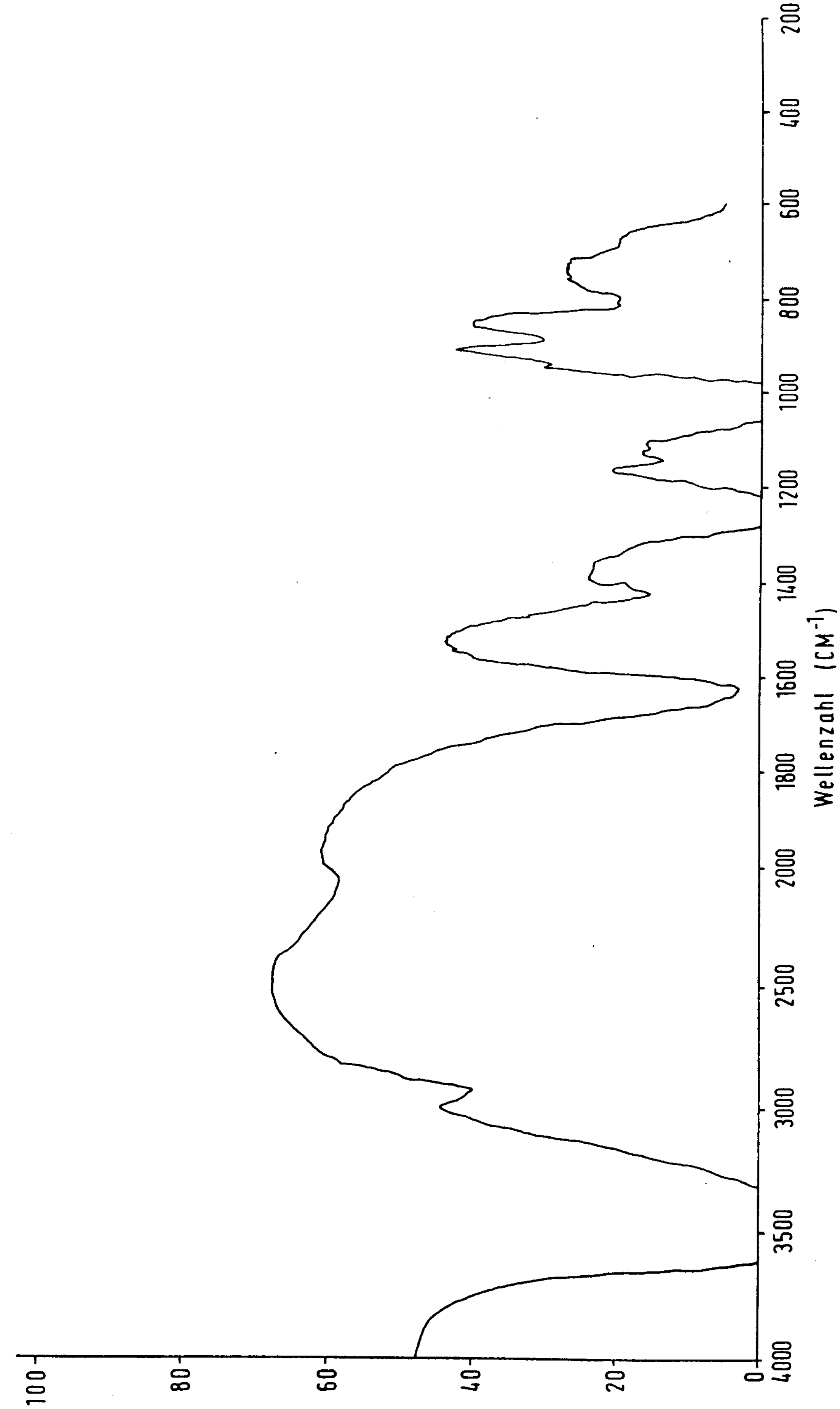

Abb.9B

$^{1}$H-NMR-Spektrum eines unfraktionierten Heparins

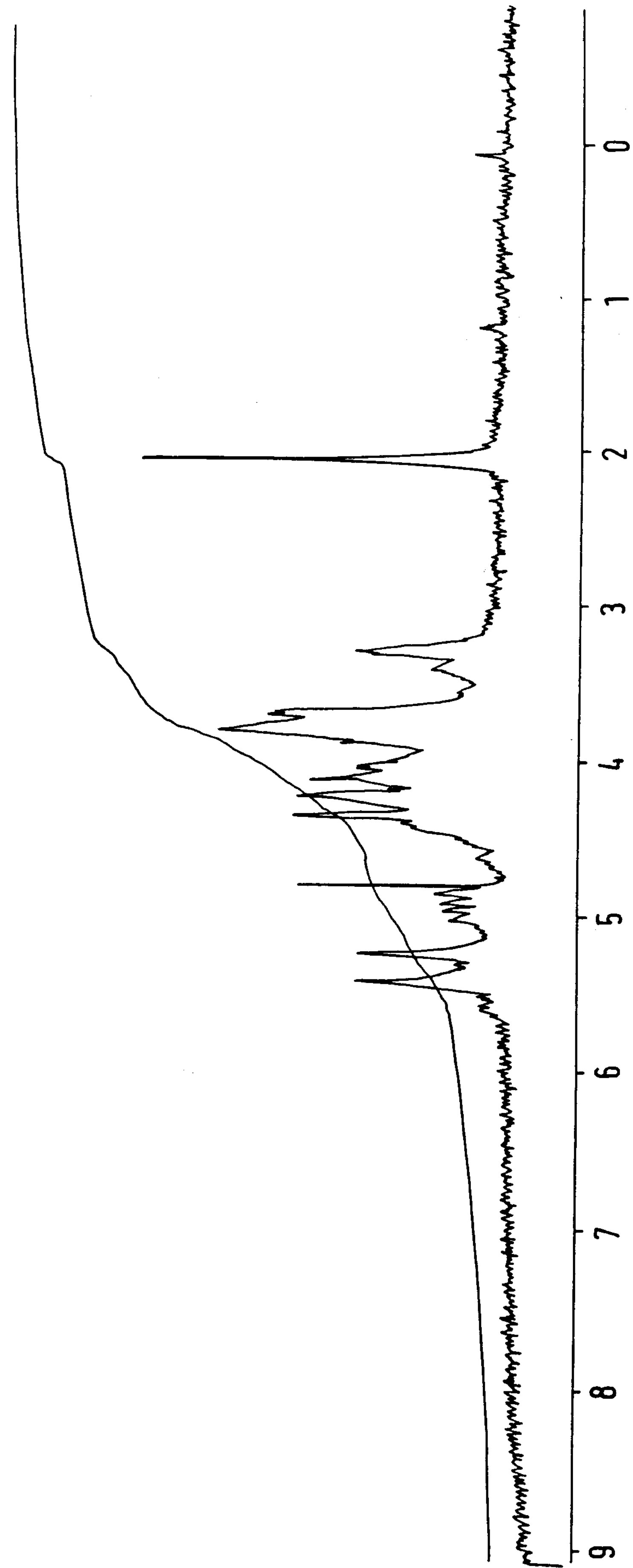

Abb.9C

$^{13}C$-NMR-Spektrum eines unfraktionierten Heparins

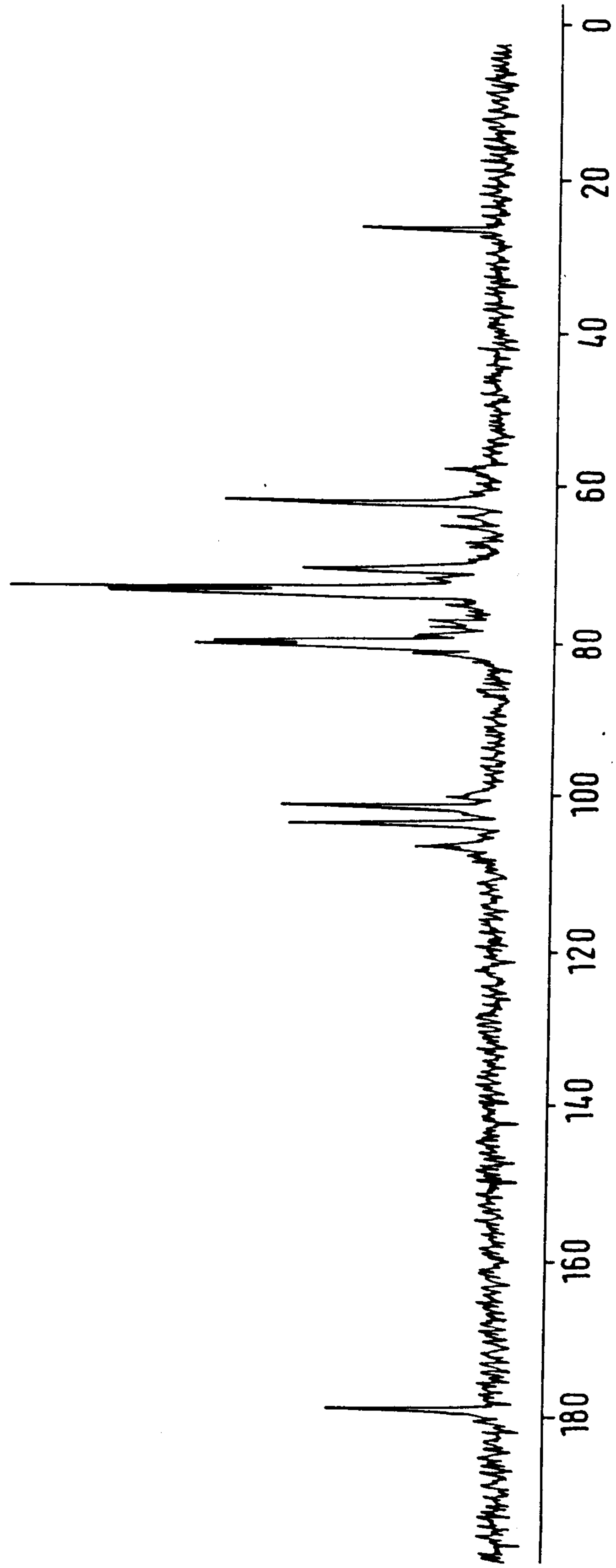

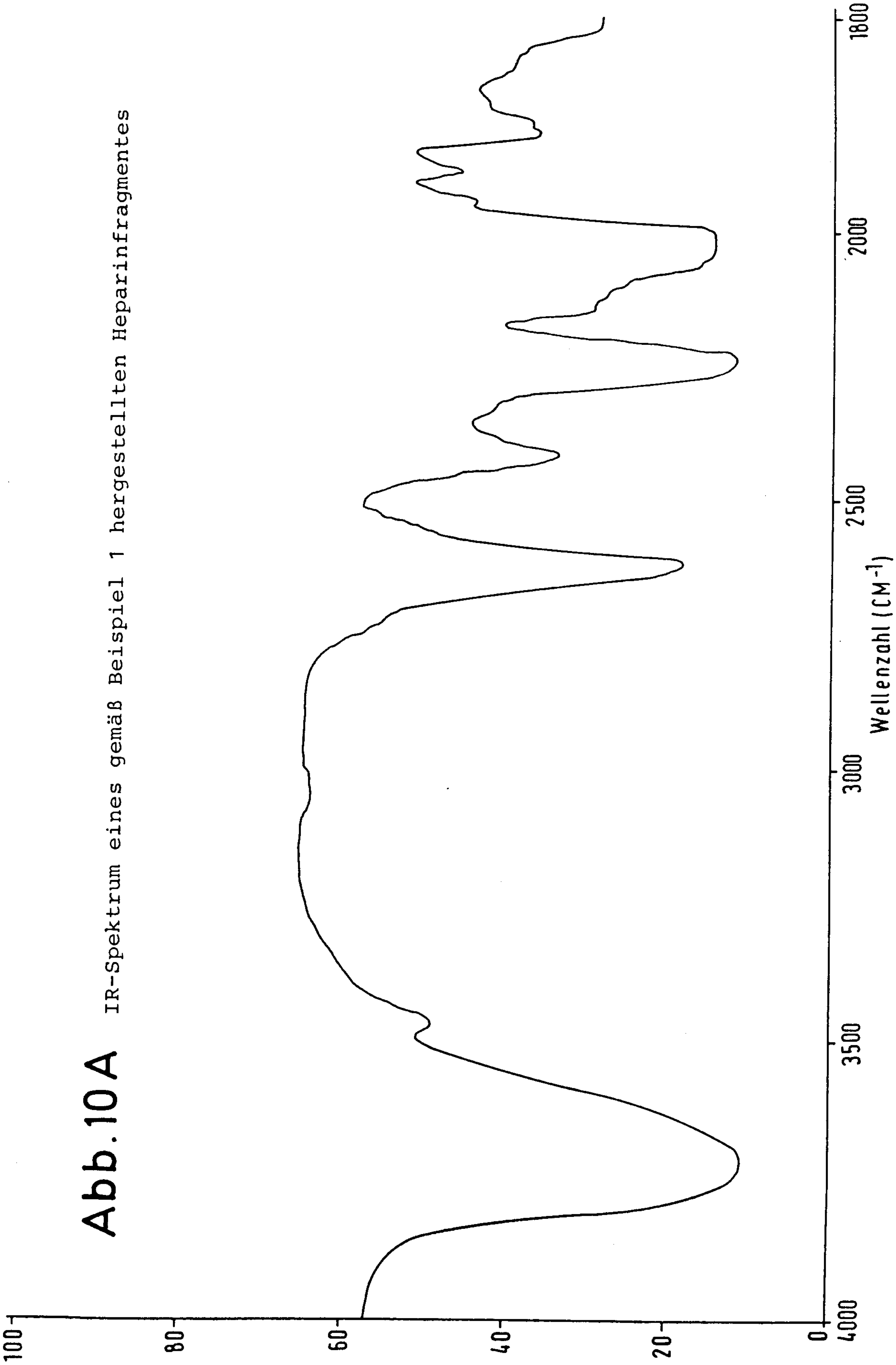


Abb.10A IR-Spektrum eines gemäß Beispiel 1 hergestellten Heparinfragmentes

# Abb.10 B

$^{1}$H-NMR-Spektrum eines gemäß Beispiel 1 hergestellten Heparinfragmentes

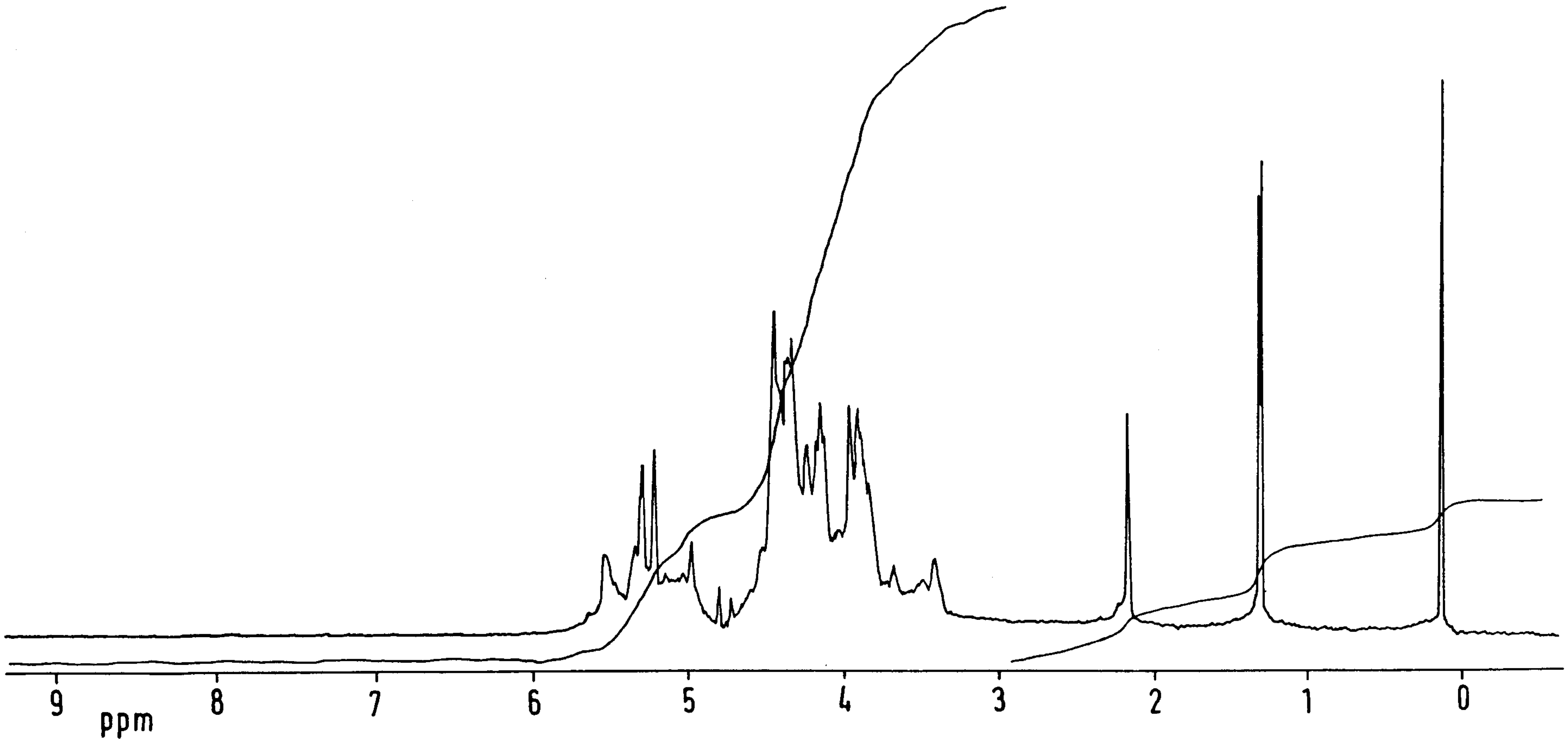

Abb.10C

$^{13}C$-NMR-Spektrum eines gemäß Beispiel 1 hergestellten Heparinfragmentes

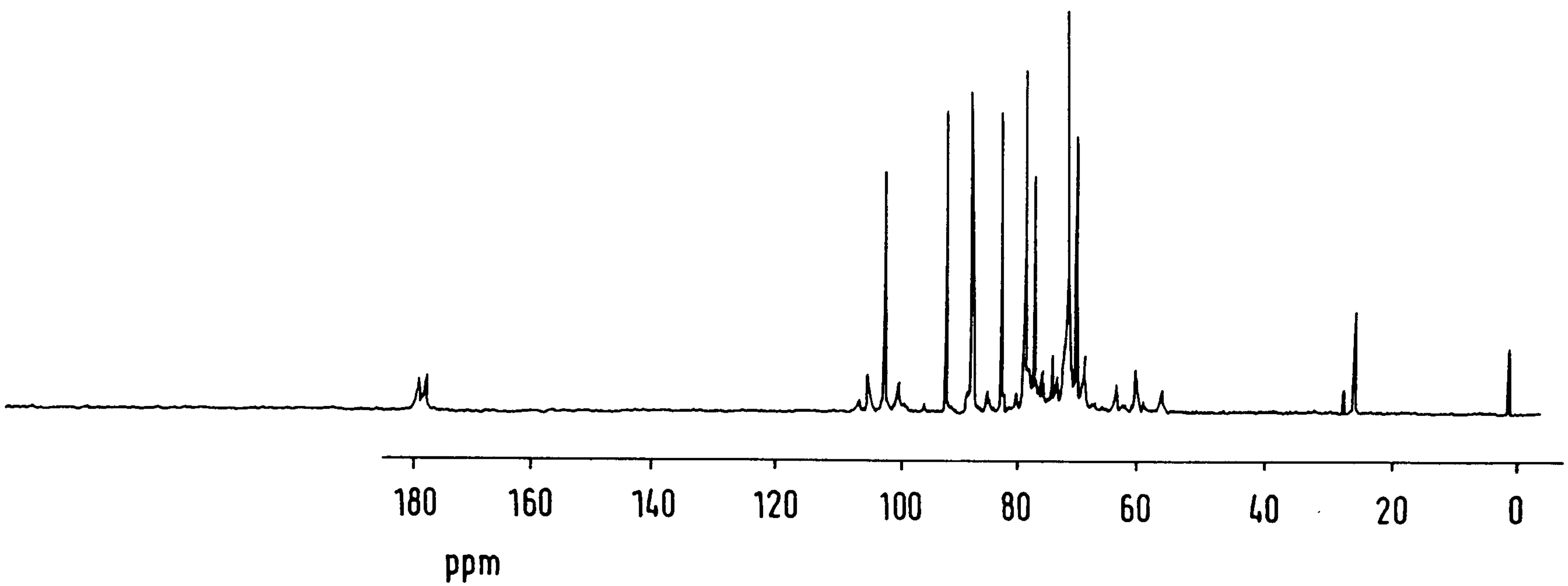

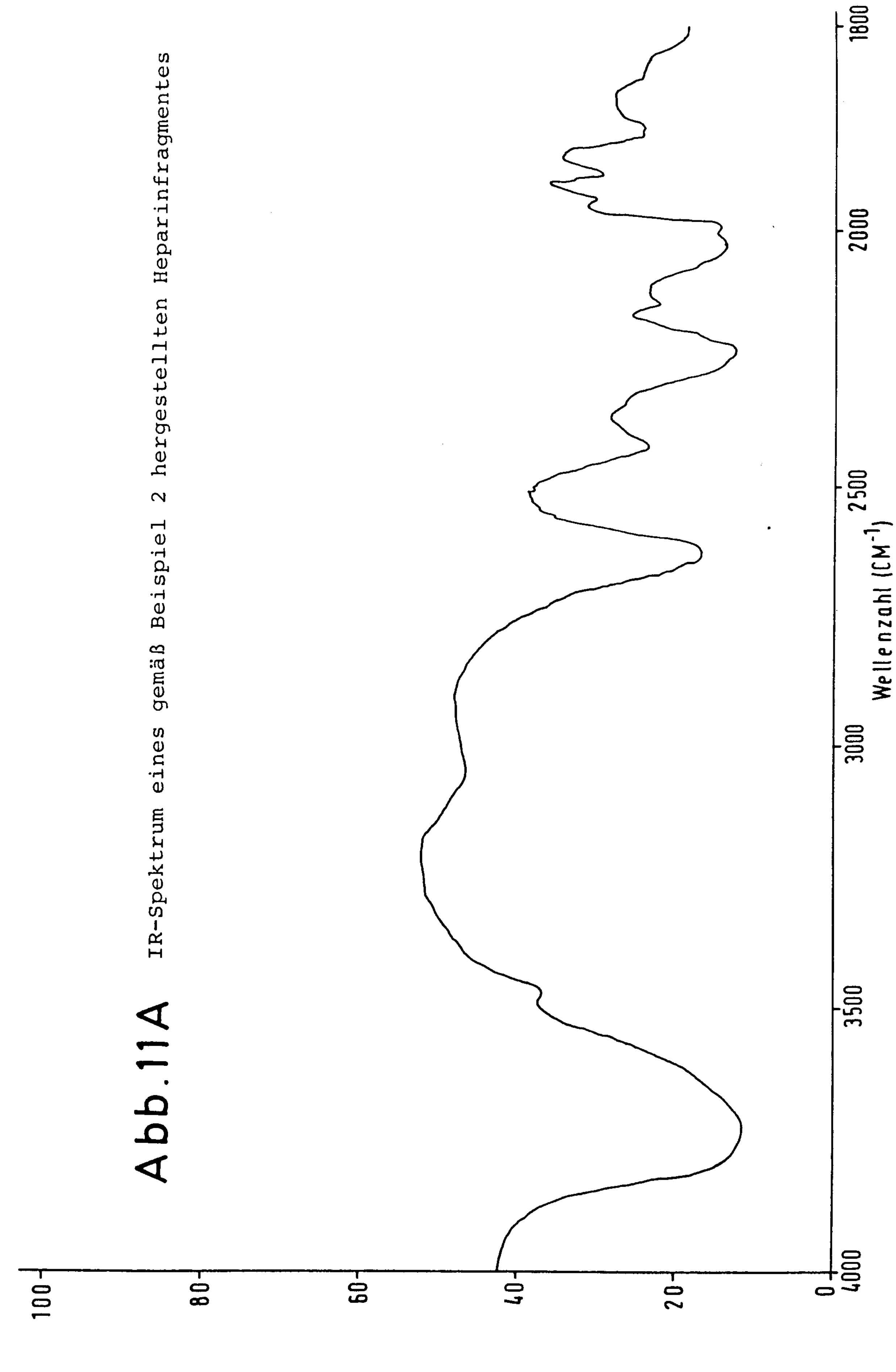


Abb.11A IR-Spektrum eines gemäß Beispiel 2 hergestellten Heparinfragmentes

# Abb.11B

$^1$H-NMR-Spektrum eines gemäß Beispiel 2 hergestellten Heparinfragmentes

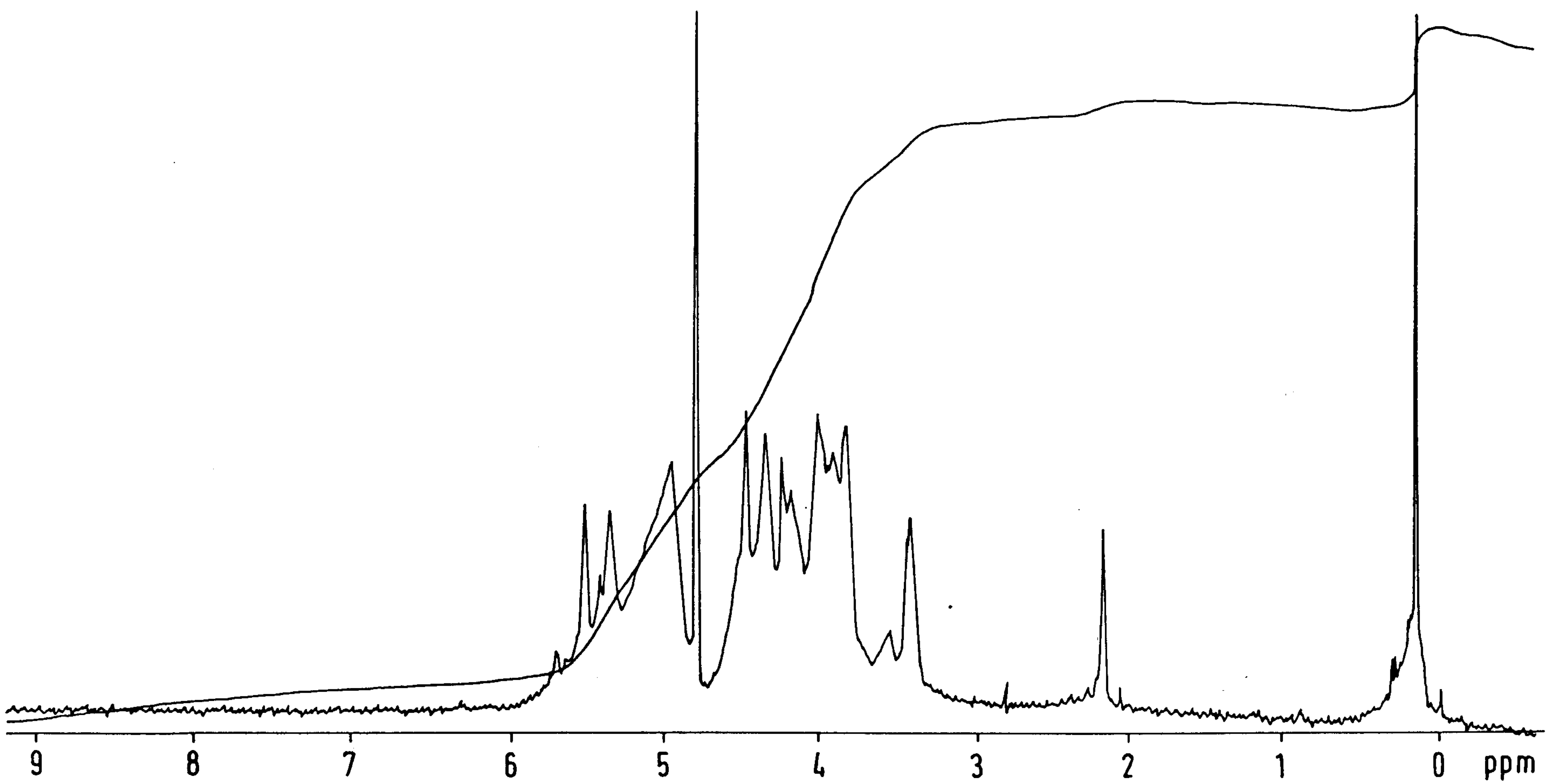

# Abb.11C

$^{13}C$-NMR-Spektrum eines gemäß Beispiel 2 hergestellten Heparinfragmentes

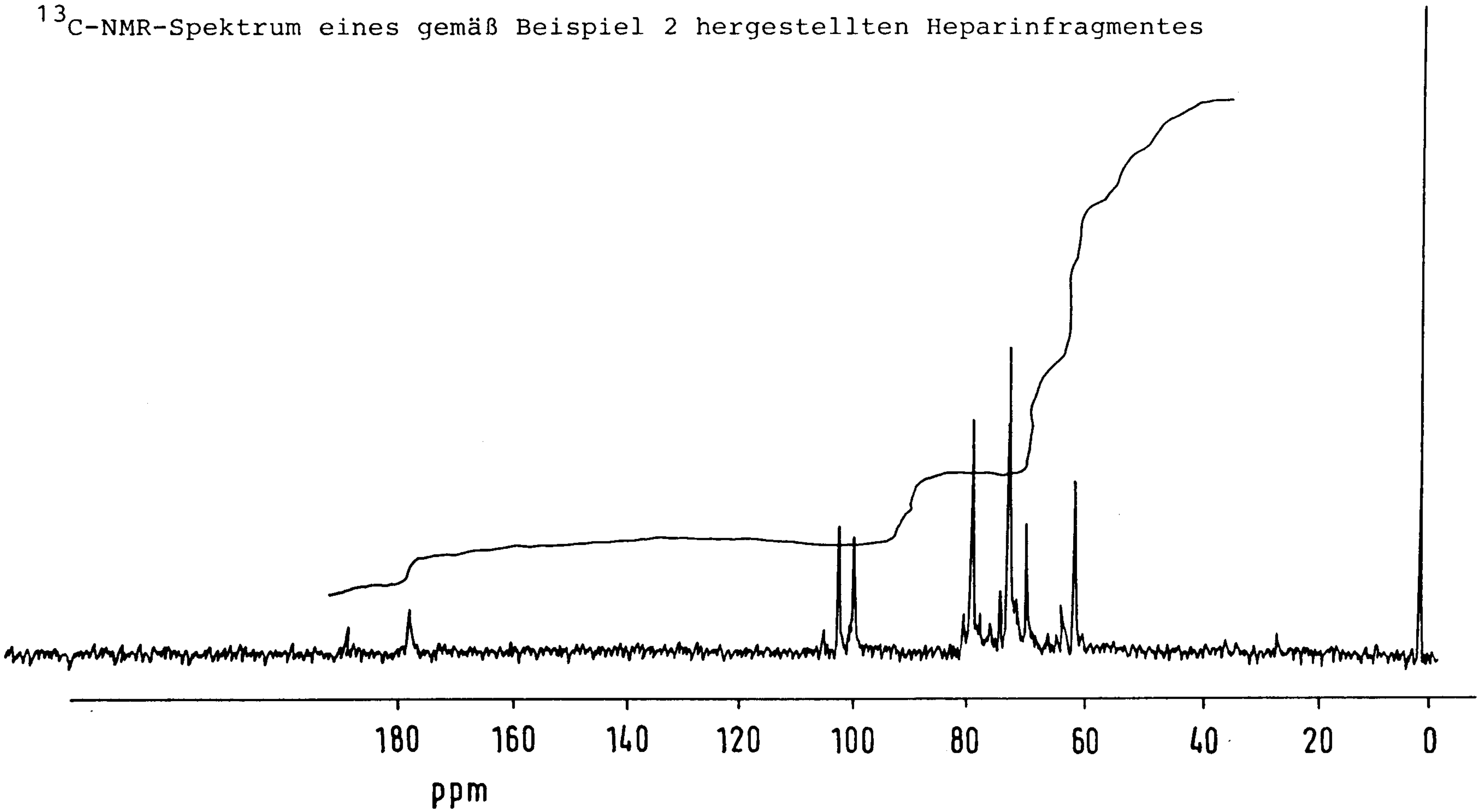

# Abb.12A

IR-Spektrum eines gemäß Beispiel 3 hergestellten Heparinfragmentes

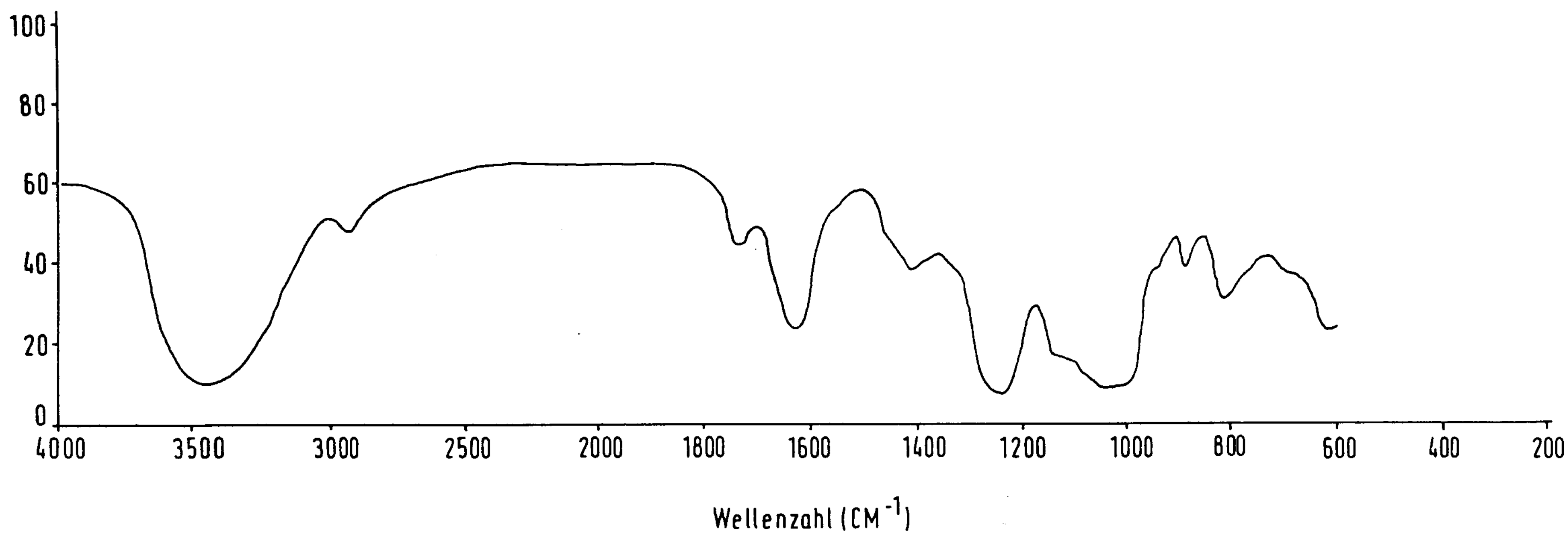

# Abb.12 B

$^1H$-NMR-Spektrum eines gemäß Beispiel 3 hergestellten Heparinfragmentes

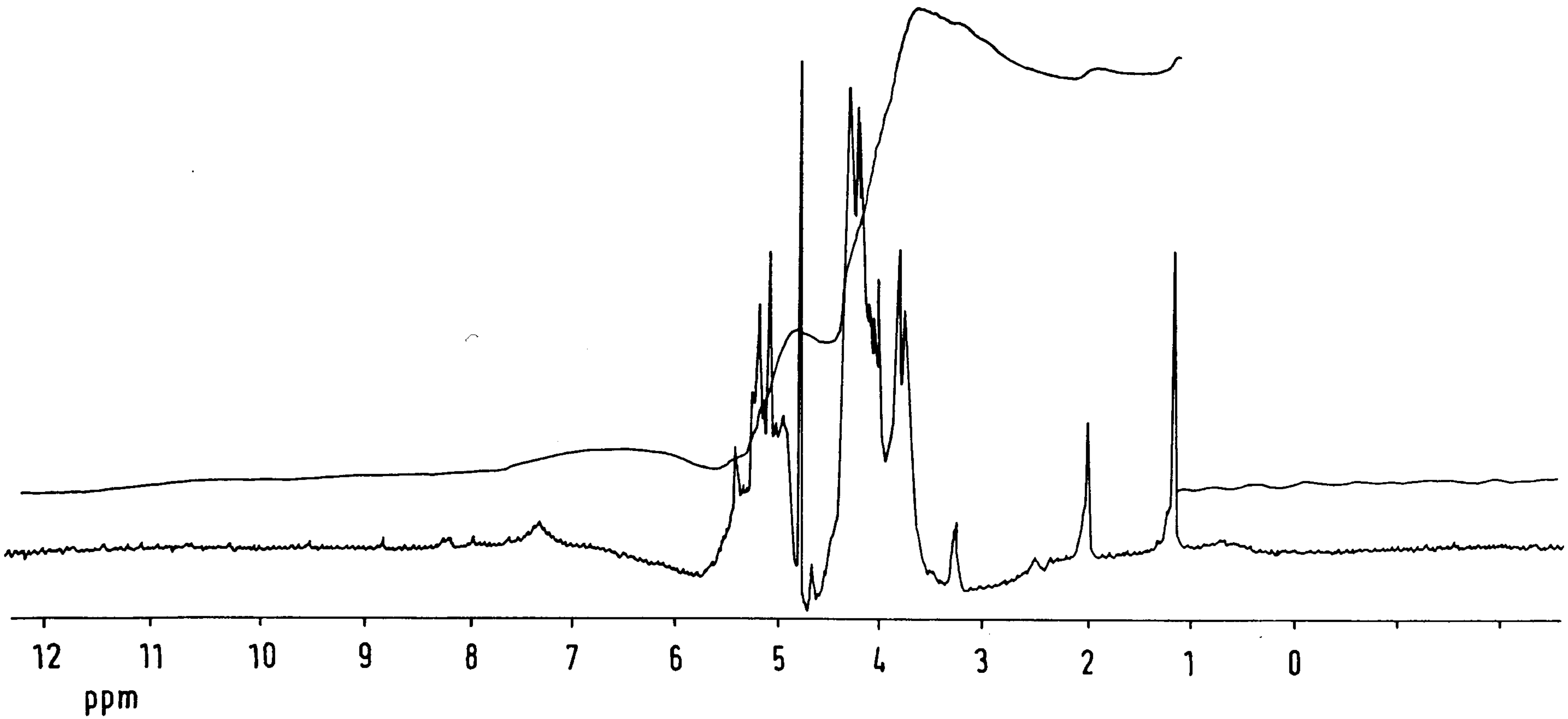

# Abb.12C

$^{13}C$-NMR-Spektrum eines gemäß Beispiel 3 hergestellten Heparinfragmentes